(19) Europäisches Patentamt
European Patent Office
Office européen des brevets

(11) Publication number: **0 357 134 B1**

# (12) EUROPEAN PATENT SPECIFICATION

(45) Date of publication of patent specification: **28.06.95**

(51) Int. Cl.[6]: **C07D 471/04**, C07D 513/04, C07D 498/04, C07D 401/14, A61K 31/495, A61K 31/425, A61K 31/42, A61K 31/505, //(C07D471/04,239:00,221:00), (C07D513/04,277:00,239:00), (C07D498/04,261:00,239:00), (C07D401/14,239:00,231:00, 211:00)

(21) Application number: **89202152.8**

(22) Date of filing: **25.08.89**

The file contains technical information submitted after the application was filed and not included in this specification

## (54) Antihypertensive 3-piperidinyl-indazole derivatives.

(30) Priority: **02.09.88 US 239915**

(43) Date of publication of application:
**07.03.90 Bulletin 90/10**

(45) Publication of the grant of the patent:
**28.06.95 Bulletin 95/26**

(84) Designated Contracting States:
**AT BE CH DE ES FR GB GR IT LI LU NL SE**

(56) References cited:
**EP-A- 0 070 053**
**EP-A- 0 089 065**
**EP-A- 0 110 435**
**EP-A- 0 135 781**
**EP-A- 0 161 498**
**EP-A- 0 184 258**
**EP-A- 0 192 935**
**EP-A- 0 219 259**
**US-A- 4 335 127**
**US-A- 4 342 870**
**US-A- 4 804 663**

(73) Proprietor: **JANSSEN PHARMACEUTICA N.V.**
**Turnhoutseweg 30**
**B-2340 Beerse (BE)**

(72) Inventor: **Vandenberk, Jan**
**Kempenlaan 15**
**B-2340-Beerse (BE)**
Inventor: **Kennis, Ludo Edmond Josephine**
**Guido Gezellestraat 50**
**B-2300-Turnhout (BE)**
Inventor: **Van Heertum, Albertus H.M.T.**
**Albertstraat 10**
**B-2350-Vosselaar (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

A number of 3-piperidinyl-1,2-benzisoxazoles and 3-piperidinyl-1,2-benzisothiazoles having antiserotonin and antipsychotic activity are known from U.S. Pat. Appl. No. 4,804,663 and J. Med. Chem. 1985, 28, 761-769. In EP-A-0,135,781, published April 3, 1985, there are disclosed a number of 3-piperidinyl-indazole derivatives as antipsychotics and analgesics. 3-Piperidinyl-indazoles having antihypertensive properties are known from EP-A-0,184,258 and EP-A-0,192,935.

The present invention is concerned with novel 3-piperidinyl-indazole derivatives having the formula

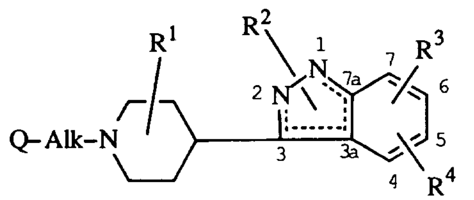

(I),

the pharmaceutically acceptable acid addition salts and stereochemically isomeric forms thereof, wherein

$R^1$ is hydrogen or $C_{1-6}$alkyl;

$R^2$ is hydrogen; $C_{1-6}$alkyl; hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl;

$R^3$ and $R^4$ each independently are hydrogen, halo, hydroxy, $C_{1-6}$alkyloxy or $C_{1-6}$alkyl;

Alk is $C_{1-4}$alkanediyl; and

Q is a bicyclic heterocyclic radical of formula

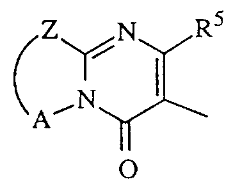

(a),

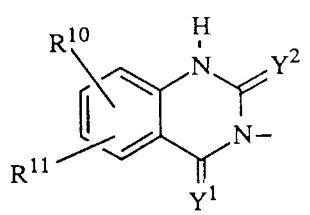

(b) or

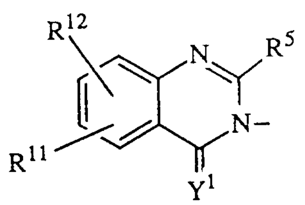

(c);

wherein

$R^5$ is hydrogen or $C_{1-6}$alkyl;

Z is -S- or $-CR^6=CR^7-$; said $R^6$ and $R^7$ each independently being hydrogen or $C_{1-6}$alkyl; orZ is $-CH_2-$ wherein one hydrogen atom may be replaced by hydroxy or $C_{1-6}$alkyl;

A is a bivalent radical $-CH_2-CH_2-$ or $-CH_2-CH_2-CH_2-$ wherein in the latter two radicals one or two hydrogen atoms may be replaced by $C_{1-6}$alkyl; or A is a bivalent radical $-CR^8=CR^9-$, wherein $R^8$ and $R^9$

each independently are hydrogen, halo, amino or $C_{1-6}$alkyl; or when Z is -S-, then A may also be $-CR^{10}=N-$, $R^{10}$ being hydrogen or $C_{1-6}$alkyl; or when Z is $-CR^6=CR^7-$, then A also may be -O- ; and

$Y^1$ and $Y^2$ each independently are O or S;

$R^{11}$ is hydrogen, halo, $C_{1-6}$alkyl, $C_{1-6}$alkyloxy, trifluoromethyl, nitro, mono- or di($C_{1-6}$alkyl)amino, $C_{1-10}$alkylcarbonylamino, cyano, $C_{1-10}$alkylcarbonyloxy, phenylmethoxy or azido;

$R^{12}$ is hydrogen or halo; and

aryl is phenyl optionally substituted with up to three substituents independently selected from $C_{1-6}$alkyl, $C_{1-6}$alkyloxy, hydroxy, halo, amino, nitro and trifluoromethyl; pyridinyl, furanyl or $C_{1-6}$alkyl substituted furanyl.

In formula (I) the dotted lines within the indazole moiety represent a conjugated diene system, its precise location depending upon the position of the $R^2$ radical : when said $R^2$ is substituted on $N^1$, then the double bonds are located between $N^2$ and position 3 and between positions 3a and 7a of the indazole system; if on the other hand, $R^2$ is substituted on $N^2$, then the double bonds are located between positions 3 and 3a and between positions 7a and $N^1$ of the indazole system.

In the foregoing definitions the term halo is generic to fluoro, chloro, bromo and iodo; $C_{1-6}$alkyl defines straight and branch chained saturated hydrocarbon radicals, having from 1 to 6 carbon atoms, such as, for example, methyl, ethyl, propyl, 1-methylethyl, butyl, 1-methylpropyl, 2-methylpropyl, 1,1-dimethylethyl, pentyl, hexyl and the like; $C_{1-10}$alkyl defines $C_{1-6}$alkyl radicals as defined hereinabove and the higher homologs thereof having from 7 to 10 carbon atoms; $C_{1-4}$alkanediyl defines bivalent straight or branch chained hydrocarbon radicals having from 1 to 4 carbon atoms, such as, for example, methylene, 1,2-ethanediyl, 1,3-propanediyl, 1,4-butanediyl and the branched isomers thereof.

The moiety Z-A in the radical of formula (b) in particular may be $-S-CH_2-CH_2-$, $-S-CH_2-CH_2-CH_2-$, $-S-CH=CH-$, $-S-CH=C(CH_3)-$, $-S-C(CH_3)=N-$, $-CH=CH-CH=CH-$, $-C(CH_3)=CH-CH=CH-$, $-CH=CH-C(CH_3)=CH-$, $-CH=CH-CCl=CH-$, $-CH=CH-CBr=CH-$, $-CH=C(CH_3)-O-$, $-CH_2-CH_2-CH_2-CH_2-$, $-CHOH-CH_2-CH_2-CH_2-$, $-CH(CH_3)-CH_2-CH_2-CH_2-$, $-CH_2-CH(CH_3)-CH_2-CH_2-$or$-CH(CH_3)-CH_2-CH(CH_3)-CH_2-$.

Depending on the nature of the various substituents the compounds of formula (I) may have several asymmetric carbon atoms . Unless otherwise mentioned or indicated, the chemical designation of compounds denotes the mixture of all possible stereochemically isomeric forms, said mixtures containing all diastereomers and enantiomers of the basic molecular structure. The absolute configuration of each chiral center may be indicated by the stereochemical descriptors R and S, this R and S notation corresponding to the rules described in Pure Appl. Chem. 1976, 45, 11-30. Stereochemically isomeric forms of the compounds of formula (I) are obviously intended to be embraced within the scope of the invention.

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, liquid chromatography and the like; and enantiomers may be separated from each other by the selective crystallization of their diastereomeric salts with optically active acids. Pure stereochemically isomeric forms may also be derived from the corresponding pure stereochemically isomeric forms of the appropriate staring materials, provided that the reaction occurs stereospecifically.

Preferred compounds within the present invention are those compounds of formula (I) wherein $R^1$ is hydrogen; and/or $R^2$ is substituted on $N^1$, and/or $R^3$ and $R^4$ each independently are hydrogen, $C_{1-6}$alkyloxy or halo; and/or Q is a radical of formula (a) wherein $R^5$ is $C_{1-6}$alkyl.

Particularly preferred compounds are those preferred compounds wherein $R^2$ is hydrogen, $C_{1-6}$alkyl, phenyl optionally sbustituted with halo or trifluoromethyl, or phenylmethyl optionally substituted with halo, $C_{1-6}$alkyloxy or trifluoromethyl; and/or $R^3$ is halo; and/or $R^4$ is hydrogen.

The most preferred compounds are those particularly preferred compounds wherein $R^3$ is 6-fluoro.

The compounds of formula (I) can generally be prepared by N-alkylating an appropriately substituted piperidine of formula (III) with an alkylating agent of formula (II).

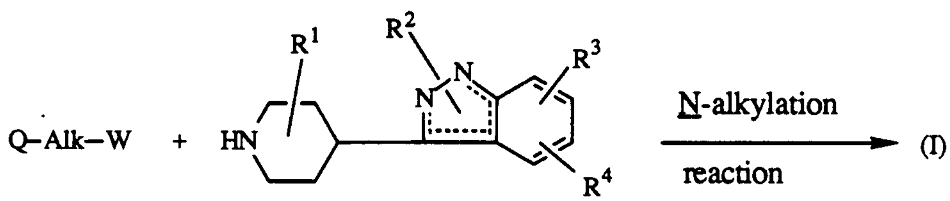


(II) (III)

In formula (II) and in any formula hereinafter wherein it occurs, W represents a reactive leaving group such as, for example, halo, e.g. chloro, bromo or iodo, or a sulfonyloxy group, e.g. methanesulfonyloxy, benzenesulfonyloxy, 4-methylbenzenesulfonyloxy and the like.

The reaction of (II) with (III) can conveniently be conducted in a reaction-inert solvent such as, for example, an aromatic hydrocarbon, e.g., benzene, methylbenzene, dimethylbenzene, and the like; a lower alkanol, e.g., methanol, ethanol, 1-butanol and the like; a ketone, e.g., 2-propanone, 4-methyl-2-pentanone and the like; an ether, 1,4-dioxane, 1,1'-oxybisethane, tetrahydrofuran and the like; N,N-dimethylformamide ; N,N-dimethylacetamide ; nitrobenzene 1,3-dimethyl-3,4,5,6-tetrahydro-2(1H)-pyrimidinone ; 1,3-dimethyl-2-imidazolidinone ; 1-methyl-2-pyrrolidinone; and the like. The addition of an appropriate base such as, for example, an alkali or an earth alkaline metal carbonate, hydrogen carbonate, hydroxide, alkoxide or hydride, e.g., sodium carbonate, sodium hydrogen carbonate, potassium carbonate, sodium hydroxide, sodium methoxide, sodium hydride and the like, or an organic base such as, for example, a tertiary amine, e.g., N,N-diethylethanamine, N-(1-methyl-ethyl)-2-propanamine, 4-ethylmorpholine and the like may be utilized to pick up the acid which is liberated during the course of the reaction. In some circumstances the addition of a iodide salt, preferably an alkali metal iodide, is appropriate. Somewhat elevated temperatures may enhance the rate of the reaction.

The compounds of formula (I) wherein $R^2$ is other than hydrogen, said $R^2$ being represented by $R^{2-a}$, and said compounds by formula (I-a), can be obtained by N-alkylating or N-acylating a 3-piperidinyl-indazole of formula (I-b) wherein $R^2$ is hydrogen, with an alkylating or acylating reagent $R^{2-a}$-$W^1$ (IV).

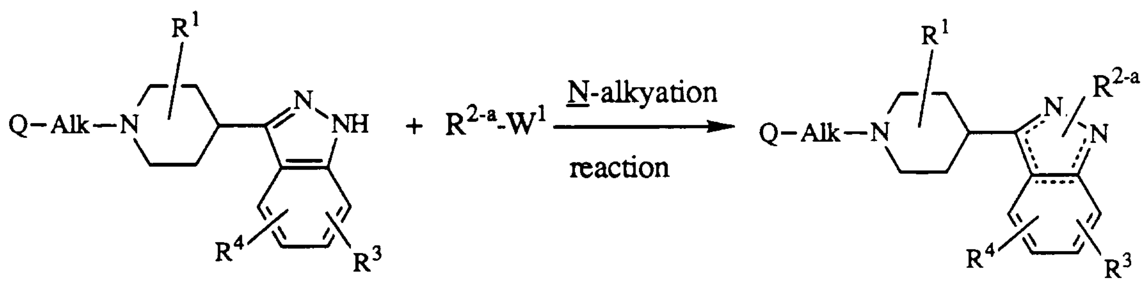


(I-b) (IV) (I-a)

In the reagent of formula (IV) $W^1$ represents an appropriate leaving group such as halo, e.g. chloro, bromo and the like; or a sulfonyloxy group, e.g. methanesulfonyloxy, 4-methylbenzenesulfonyloxy and the like; in the instances wherein $R^{2-a}$ is $C_{1-6}$alkylcarbonyl or optionally substituted phenylcarbonyl $W^1$ may also represent respectively $C_{1-6}$alkylcarbonyloxy or optionally substituted phenylcarbonyloxy (i.e. $R^{2-a}$-$W^1$ is an acid anhydride).

Said N-alkylation reaction of (I-b) with a reagent (IV) may be carried out by stirring and, if desired, heating the reactants, optionally in an appropriate reaction-inert solvent such as, for example, an aromatic hydrocarbon, e.g., benzene, methylbenzene, dimethylbenzene, and the like; a lower alkanol, e.g., methanol, ethanol, 1-butanol and the like; a ketone, e.g., 2-propanone, 4-methyl-2-pentanone and the like; an ether, 1,4-dioxane, 1,1'-oxybisethane, tetrahydrofuran and the like ; a dipolar aprotic solvent, e.g. N,N-dimethylformamide, N,N-dimethylacetamide, nitrobenzene, 1,3-dimethyl-3,4,5,6-tetrahydro-2(1H)-pyrimidinone, 1,3-dimethyl-2-imidazolidinone, 1-methyl-2-pyrrolidinone and the like. Further the addition of a base such as, for example, aqueous alkali, e.g. sodium or potassium hydroxide, sodium or potassium carbonate and the like ; or an alkali metal alkoxide such as, for example, sodium methoxide, sodium ethoxide, potassium tert.-butoxide and the like may be appropriate. In case mixtures of 1- and 2-substituted indazoles are obtained, said mixtures may be separated by the application of art-known methods such as, for example, selective crystallization, chromatography and the like.

The compounds of formula (I) may also be prepared by the cyclization reaction of an intermediate of formula (V) with an appropriate hydrazine derivative $R^2$-NH-$NH_2$ (VI) or an acid addition salt thereof.

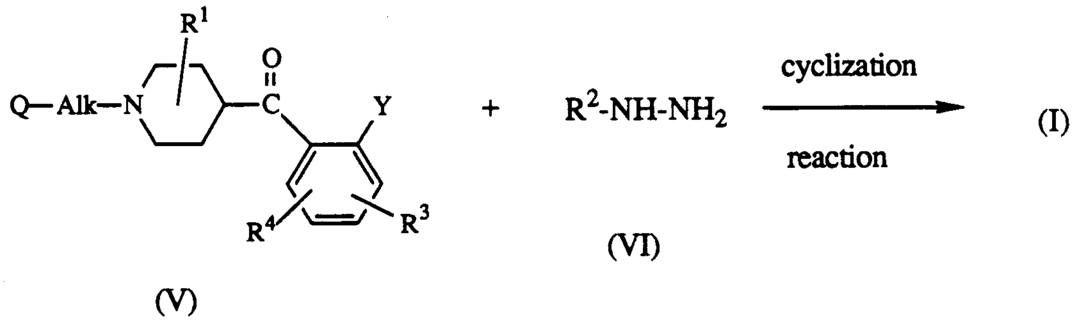


(V) (VI) (I)

In formula (V) Y represents an appropriate leaving group such as, for example, halo, e.g. fluoro or chloro; or a nitro group. Said cyclization reaction may be conducted by stirring, and if desired, heating the reactants in a suitable reaction-inert solvent in the presence of an appropriate base. Suitable solvents generally have a relatively high boiling point and are, for example, water; alkanols, e.g. methanol, ethanol, 1-butanol and the like; diols, e.g. 1,2-ethanediol and the like; aromatic hydrocarbons, e.g. benzene, methylbenzene, dimethylbenzene and the like; halogenated hydrocarbons, e.g. trichloromethane, tetrachloromethane and the like; ethers, e.g. tetrahydrofuran, 1,4-dioxane, 1,1'-oxybisbutane, 1,1'-oxybis-(2-methoxyethane) and the like; dipolar aprotic solvents, e.g. N,N-dimethyl-formamide, N,N-dimethylacetamide, dimethylsulfoxide and the like; or mixtures of such solvents. Appropriate bases preferably are alkali or earth alkaline metal carbonates or hydrogen carbonates such as, for example, sodium hydrogen carbonate, sodium carbonate, potassium carbonate and the like; or amines such as N,N-diethylethanamine, 4-ethylmorpholine, N-(1-methylethyl)-2-propanamine and the like. Additionally, the compounds of formula (I) may be prepared by the nitrosation of an intermediate aniline of formula

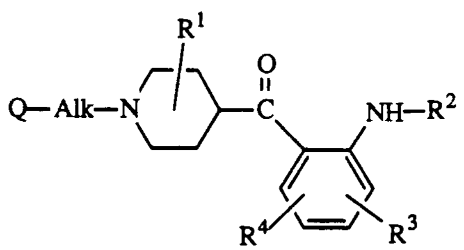 (VII),

with an alkali metal nitrite, e.g. sodium nitrite, in an aqueous acidic medium and treating the thus obtained N-nitroso compound (VIII-a) or, in case $R^2$ is hydrogen, the diazonium salt (VIII-b),

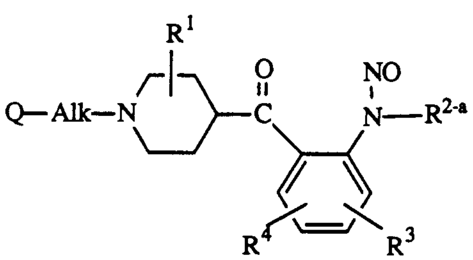

(VIII-a)

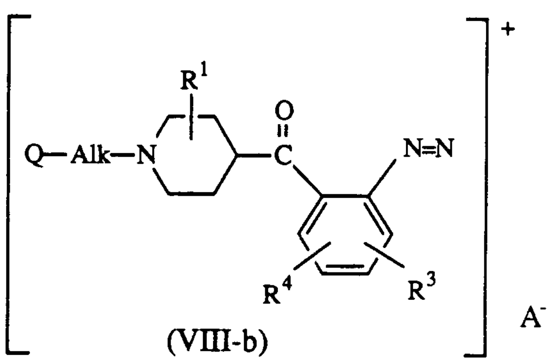

(VIII-b)

wherein $A^-$ represents the conjugated base of the acid used hereinabove, with an appropriate reducing agent such as, for example, hydrogen in the presence of a hydrogenation metal catalyst, e.g. Raney nickel or Raney cobalt ; or a sulfite, e.g. sodium sulfite, thus yielding the corresponding hydrazine derivative of formula

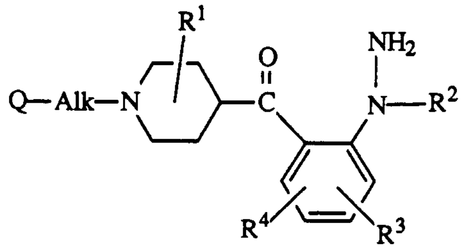

(IX),

which in most instances spontaneously, or if necessary upon increasing the temperature, may cyclize to a compound of formula (I).

The compounds of formula (I) may also be prepared following art-known procedures for building up radicals of formula Q. For example, the compounds of formula (I) wherein Q is radical of formula (b), said compounds being represented by formula (I-c), can be obtained from an intermediate of formula (X) by condensation with a reagent of formula (XI), wherein each L independently represents an appropriate leaving group.

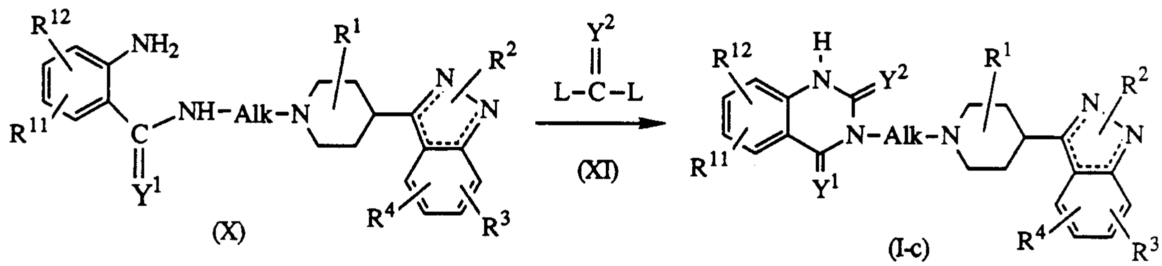

(X) (XI) (I-c)

As typical examples of reagents of formula (XI) there may be mentioned urea, thiourea, carbonic dichloride, 1,1'-carbonylbis[1H-imidazole], di($C_{1-6}$alkyl)carbonates such as dimethyl carbonate, diethylcarbonate and the like, chloroformates such methyl chloroformate, ethyl chloroformate, phenyl chloroformate and the like reagents.

The compounds of formula (I-c) can also be prepared by cyclizing an intermediate of formula (XII) with a primary amine of formula (XIII)

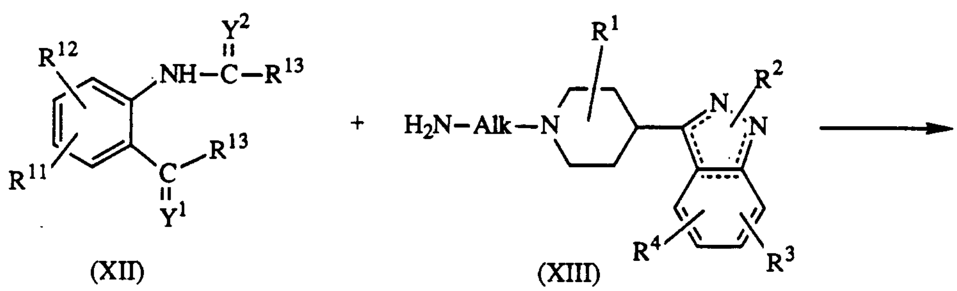

(XII) (XIII) (I-c),

or by cyclizing an isocyanate or isothiocyanate of formula (XIV) with an amine of formula (XIII).

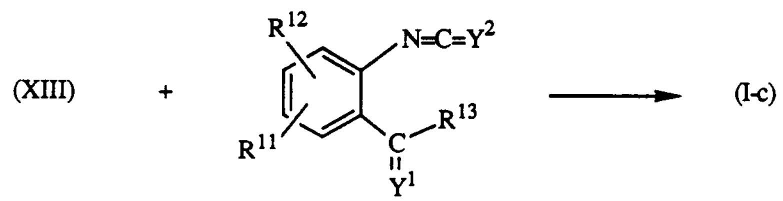

(XIV)

In the foregoing reaction schemes each $R^{13}$ independently represents an appropriate leaving group such as, for example, $C_{1-6}$alkyloxy, amino, mono- or di($C_{1-6}$alkyl)amino; and in formula (XII) both $R^{13}$ groups taken together may also represent - O-. Said cyclization reactions are conveniently conducted by stirring and, if desired, heating the reactants, optionally in a suitable reaction-inert solvent such as an aliphatic or aromatic hydrocarbon, e.g. petroleum ether, dimethylbenzene and the like ; a halogenated hydrocarbon, e.g. dichloromethane, trichloromethane, 1,2-dichloroethane and the like ; an ether, e.g. tetrahydrofuran, 1,4-dioxane and the like ; a ketone, e.g. 2-propanone, 4-methyl-2-pentanone and the like.

The compounds of formula (I) wherein Q is a radical of formula (c), said compounds being represented by formula (I-d), can be obtained by treating an intermediate of formula (X) with a carboxylic acid of formula (XV) or, a suitable functional derivative thereof such as an acyl halide, an anhydride or an ester.

In (XV) and in the formulae hereinafter each $R^{14}$ independently represents an appropriate leaving group such as, for example, hydroxy, halo, $C_{1-6}$alkyloxy, $C_{1-6}$alkylcarbonyloxy, amino, mono- or di-( $C_{1-6}$alkyl)-amino.

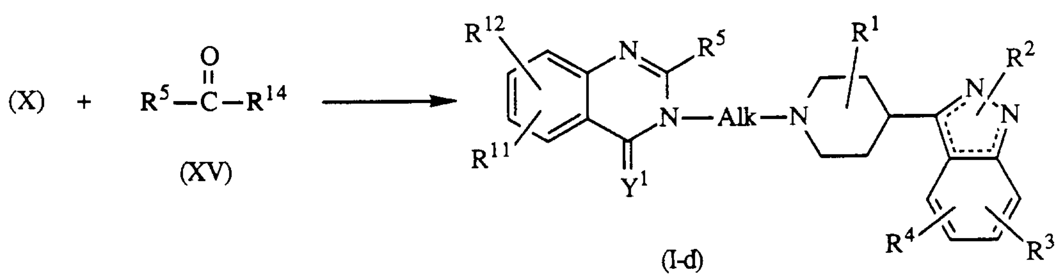

(XV)

(I-d)

The compounds of formula (I) wherein Q is a radical of formula (a), said compounds being represented by the formula (I-e), can be prepared following art-known cyclization procedures for preparing pyrimidin-4-ones such as, for example, by reacting an amine of formula (XVI) with a $\beta$-dicarbonyl compound of formula (XVII) or by cyclizing a reagent of formula (XVIII) with an enamine of formula (XIX).

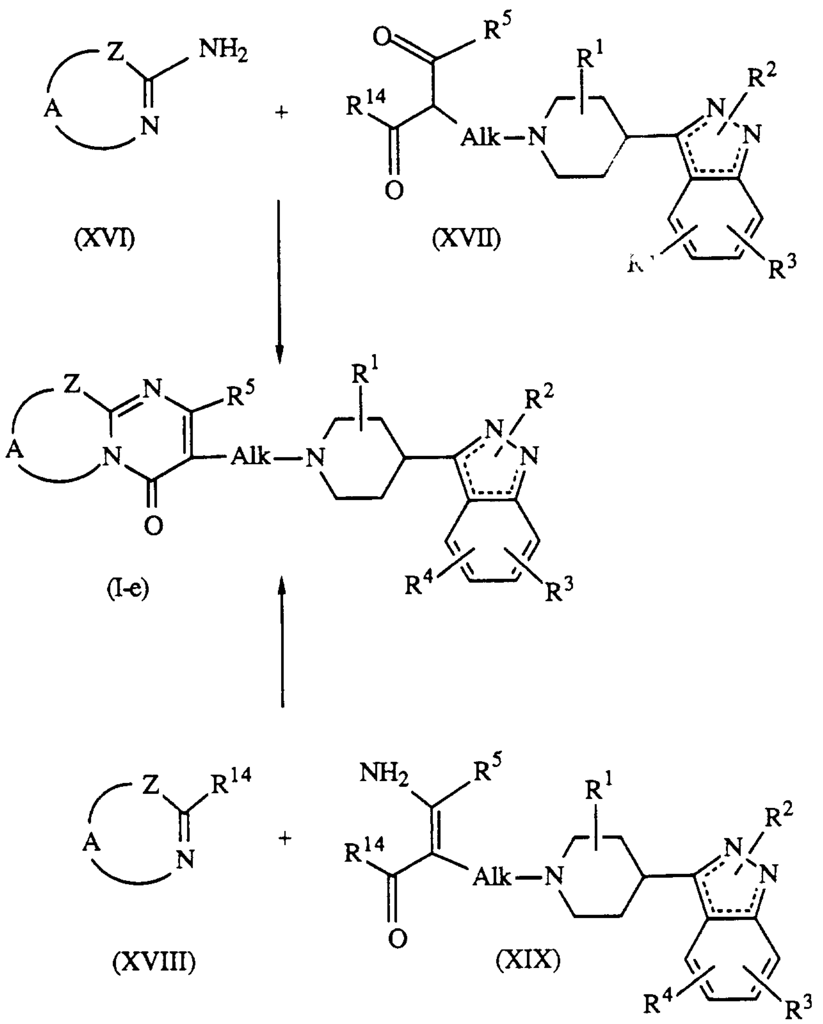

(XVI) + (XVII) → (I-e) ← (XVIII) + (XIX)

Said cyclization reactions may generally be carried out by stirring the reactants, optionally in the presence of a suitable reaction-inert solvent such as, for example, an aliphatic, alicyclic or aromatic hydrocarbon, e.g., hexane, cyclohexane, benzene and the like; or pyridine, N,N-dimethylformamide and the like dipolar aprotic solvents. Elevated temperatures may be appropriate to enhance the reaction rate ; more in particular it may be advantageous to carry out the reaction at the reflux temperature of the reaction mixture.

Following the same procedure the compounds of formula (I-e) can also be prepared by cyclizing an intermediate of formula (XIX) with a reagent of formula (XX).

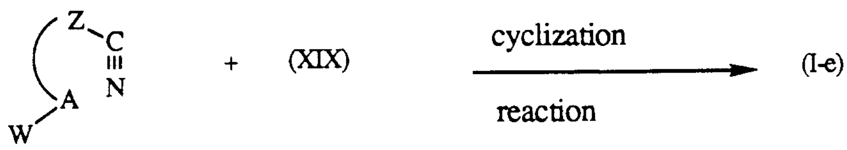


+ (XIX) (I-e)

(XX)

The compounds of formula (I-e) wherein Z is S, said compounds being represented by the formula (I-e-1), can also be prepared by cyclizing a 2-mercaptopyrimidinone of formula (XXI) with a reagent of formula (XXII).

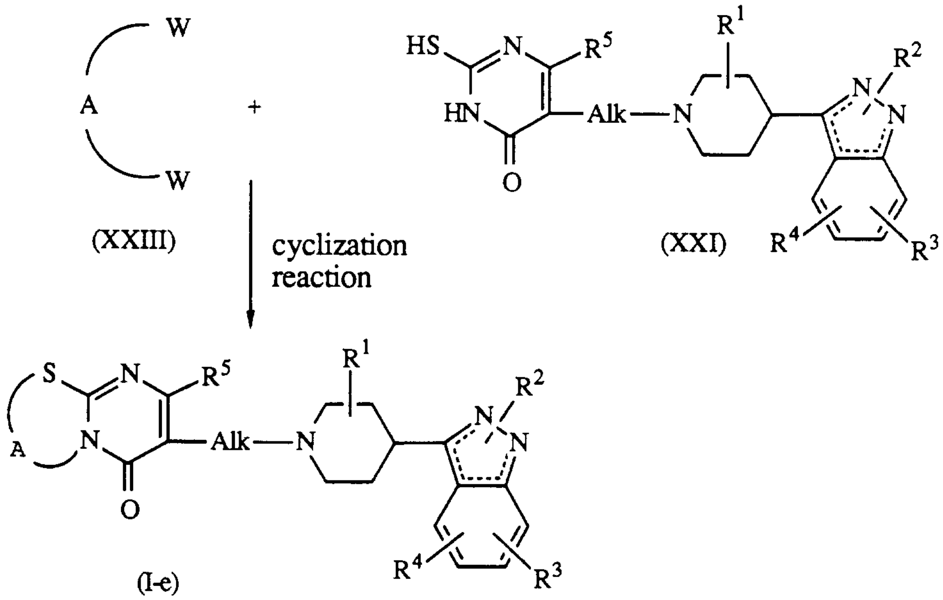


(XXIII) (XXI) (I-e)

The compounds of formula (I-e-1) wherein A is $CR^8=CR^9$, said compounds being represented by the formula (I-e-2), can be prepared by cyclizing a 2-mercaptopyrimidinone of formula (XXI) with a reagent of formula (XXIII).

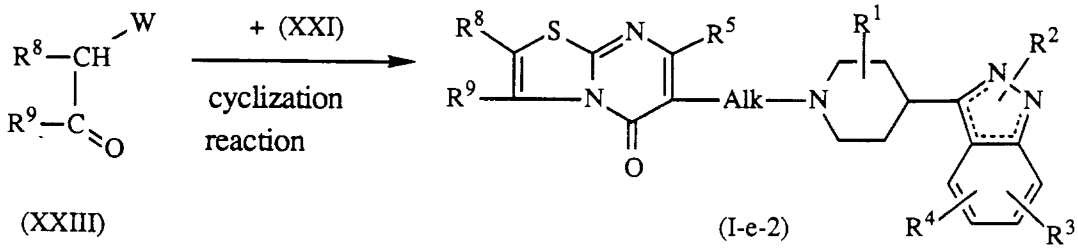


(XXIII) (I-e-2)

Said cyclization reactions for preparing the compounds of formulae (I-e-1) and (I-e-2) may generally be carried out by stirring the reactants, if desired, in the presence of a suitable reaction-inert solvent such as, for example, an aliphatic, alicyclic or aromatic hydrocarbon, e.g., hexane, cyclohexane, benzene and the like; or pyridine N,N-dimethylformamide and the like dipolar aprotic solvents. Elevated temperatures may be appropriate to enhance the reaction-rate, more in particular it may be preferred to carry out the reaction at the reflux temperature of the reaction mixture.

The compounds of formula (I) may also be converted into each other following art-known functional group transformation procedures.

The compounds of formula (I) have basic properties and, consequently, they may be converted to their therapeutically active non-toxic acid addition salt forms by treatment with appropriate acids, such as, for example, inorganic acids, such as hydrohalic acid, e.g. hydrochloric, hydrobromic acid and the like, sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methyl-benzenesulfonic, cyclohexanesulfamic, 2-hydroxybenzoic, 4-amino-2-hydroxybenzoic and the like acids. Conversely the salt form can be converted into the free base form by treatment with alkali. The term acid addition salt as used hereinabove also comprises the solvates which the compounds of formula (I) are able to form and said solvates are meant to be included within the scope of the present invention. Examples of such solvates are e.g., the hydrates, alcoholates and the like.

A number of intermediates and starting materials in the foregoing preparations are known compounds which may be prepared according to art-known methodologies of preparing said or similar compounds. The intermediates of formula (II) and their preparations are described in U.S. Patent Nos. 4,335,127, 4,342,870,

4,443,451 and 4,485,107, and in the references cited therein. The intermediates of formula (III) are known from EP-A-0,135,781.

The compounds of formula (I), the pharmaceutically acceptable acid addition salts and stereochemically isomeric forms thereof, are potent antagonists of neurotransmitters and in particular of the mediators serotonin and dopamine. Antagonizing said mediators will suppress or relieve a variety of symptoms associated with phenomena induced by the release, in particular the excessive release, of these mediators. Therapeutic indications for using the present compounds are mainly in the CNS area, the gastrointestinal and cardiovascular field and related domains. Serotonin antagonists are reportedly effective in combatting psychoses, aggressive behaviour, anxiety, depression and migraine. Combined serotonin-dopamine antagonists are especially interesting as they appear to offer relief of both the positive and negative symptoms of schizophrenia. Therapeutic applications in the gastrointestinal field comprise their use as, for instance, antidiarrhoeals, inhibitors of gastro-oesophageal reflux and particularly antiemetics, e.g. in cancer patients receiving chemotherapy and radiation treatment. Further, serotonin is a potent broncho- and vasoconstrictor and thus the present antagonists may be used against hypertension and vascular disorders. In addition, serotonin antagonists have been associated with a number of other properties such as, the suppression of appetite and promotion of weight loss, which may prove effective in combatting obesity; and also the alleviation of withdrawal symptoms in addicts trying to discontinue drinking and smoking habits.

The compounds of the instant invention are particularly useful as antihypertensive agents because of their ability to depress significantly both systolic and diastolic blood pressure in warm-blooded animals. The antihypertensive effect of the instant compounds is evidenced in the "Blood pressure lowering effect in spontaneous hypertensive rats" test. Particularly interesting are also the observations made in the combined "Apomorphine, tryptamine and norepinephrine in rats" test: contrary to most structurally related 3-piperidinyl benzazoles, the instant compounds do not show significant activity on the central nervous system, but rather act peripherally.

In view of their useful pharmacological properties, the subject compounds may be formulated into various pharmaceutical forms for administration purposes. To prepare the pharmaceutical compositions of this invention, an effective amount of the particular compound, in acid addition salt or base form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoon fuls and the like, and segregated multiples thereof.

In view of the usefulness of the subject compounds in the treatment of hypertension it is evident that the present invention provides a method of treating warm-blooded animals suffering from hypertension, said method comprising the systemic administration of a pharmaceutically effective amount of a compound of

formula (I) or a pharmaceutically acceptable acid addition salt thereof in admixture with a pharmaceutical carrier. Those of skill in the treatment of hypertension could easily determine the effective amount from the test results presented here. In general it is contemplated that an effective amount would be from 0.01 mg/kg to 4 mg/kg body weight, more preferably from 0.04 mg/kg to 2 mg/kg body weight per day.

It is evident that said effective amount may be lowered or increased depending on the response of the treated subject and/or depending on the evaluation of the physician prescribing the compounds of the instant invention. The effective amount ranges mentioned hereinabove are therefore guidelines only and are not intended to limit the scope or use of the invention to any extent.

The following examples are intended to illustrate and not to limit the scope of the present invention. Unless otherwise stated all parts therein are by weight.

## EXPERIMENTAL PART

### A. Preparation of Intermediates

#### Example 1

a) To a stirred mixture of 15.8 parts of a sodium hydride dispersion 50% and 660 parts of dimethyl sulfoxide were added portionwise 78.4 parts of 1-acetyl-4-(6-fluoro-2H-indazol-3-yl)piperidine under nitrogen atmosphere. Upon complete addition, stirring was continued for 1 hour at room temperature. 42 Parts of chloromethylbenzene were added dropwise during 45 minutes. Upon completion, stirring was continued overnight at room temperature. The reaction mixture was poured into crushed ice and the product was extracted with dichloromethane. The extract was dried, filtered and evaporated. The residue was purified by column chromatography (HPLC) over silica gel using a mixture of methylbenzene and methanol (90:10 by volume) as eluent. The first fraction was collected and the eluent was evaporated, yielding 91 parts (86.3%) of 1-acetyl-4-[6-fluoro-2-(phenylmethyl)-2H-indazol-3-yl]piperidine as a residue (int. 1).

b) A mixture of 8 parts of 1-acetyl-4-[6-fluoro-2-(phenylmethyl)-2H-indazol-3-yl]-piperidine and 70 parts of a hydrochloric acid solution 6N was stirred for 6 hours at reflux temperature. The reaction mixture was evaporated and the oily residue was crystallized from 2-propanol. The product was filtered off and dried, yielding 2 parts (25.1%) of 6-fluoro-2-(phenylmethyl)-3-(4-piperidinyl)-2H-indazole monohydrochloride; mp. >300 °C (int. 2).

#### Example 2

a) A mixture of 50 parts of 5-methyl-3-isoxazolamine, 70 parts of 3-acetyl-4,5-dihydro-2(3H)-furanone, 435 parts of methylbenzene and 16 parts of polyphosphoric acid was stirred and refluxed for 3 hours using a water separator. The reaction mixture was concentrated in vacuo, yielding 99 parts (95.1%) of 4,5-dihydro-3-[1-(5-methyl-3-isoxazolyl)imino]ethyl]-2(3H)-furanone as an oily residue (int. 3).

b) To a stirred mixture of 98 parts of 4,5-dihydro-3-[1-(5-methyl-3-isoxazolyi)imino]ethyl]-2(3H)-furanone, 348 parts of methylbenzene and 300 parts of trichloromethane were added dropwise 150 parts of phosphoryl chloride. Upon complete addition, stirring was continued for 3 hours at reflux temperature. The reaction mixture was concentrated to half its volume and the residue was poured into crushed ice. The whole was treated with an ammonium hydroxide solution and the product was extracted twice with 240 parts of 4-methyl-2-pentanone. The combined extracts were dried, filtered and evaporated in vacuo. The residue was dissolved in trichloromethane, filtered over silica gel and the filtrate was concentrated in vacuo. The residue was crystallized form a mixture of methylbenzene and 2,2'-oxybispropane, yielding 96 parts (88.2%) of 6-(2-chloroethyl)-2,5-dimethyl-7H-isoxazolo[2,3-a]pyrimidin-7-one; mp. 165 °C (int. 4).

### B. Preparation of Final Compounds

#### Example 3

A mixture of 4 parts of 3-(2-chloroethyl)-2,4(1H,3H)-quinazolinedione, 4.4 parts of 6-fluoro-1-methyl-3-(1-piperidinyl)-1H-indazole monohydrochloride, 10 parts of sodium carbonate, 0. 1 parts of potassium iodide and 144 parts of 4-methyl-2-pentanonewas stirred overnight at reflux temperature. After cooling, the reaction mixture was poured into water. The separated organic layer was dried, filtered and evaporated. The residue was purified by column chromatography over silica gel using a mixture of trichloromethane and methanol

(95:5 by volume) as eluent. The pure fractions were collected and the eluent was evaporated. The residue was crystallized from acetonitrile. The product was filtered off and dried, yielding 4 parts (59.3%) of 3-[2-[4-(6-fluoro-1-methyl-1H-indazol-3-yl)-1-piperidinyl]ethyl]-2,4(1H,3H)quinazolinedione; mp. 250.1 °C (compound 1).

Example 4

A mixture of 4.4 parts of 6-(2-bromoethyl)-2,3-dihydro-7-methyl-5H-thiazolo[3,2-a]pyrimidin-5-one monohydrobromide, 3.7 parts of 6-fluoro-3-(4-piperidinyl)-1H-indazole dihydrochloride, 4.25 parts of sodium carbonate and 120 parts of 4-methyl-2-pentanone was stirred for 6 hours at reflux temperature. After cooling, the reaction mixture was filtered. The precipitate was stirred in water and the whole was filtered again. The precipitated product was purified by column chromatography over silica gel using a mixture of trichloromethane and methanol (90:10 by volume) as eluent. The pure fractions were collected and the eluent was evaporated. The residue was crystallized from 2-propanol. The product was filtered off and dried, yielding 2.5 parts (48.3%) of 6-[2-[4-(6-fluoro-1H-indazol-3-yl)-1-piperidinyl]ethyl]-2,3-dihydro-7-methyl-5H-thiazolo[3,2-a]pyrimidin-5-one; mp. 207.6 °C (compound 2).

Example 5

0.7 Parts of a sodium hydride dispersion 50% in mineral oil were suspended in petroleum ether under a nitrogen atmosphere. The solvent was decanted and 55 parts of dimethyl sulfoxide were added. 6 Parts of 6-[2-[4-(6-fluoro-1H-indazol-3-yl)-1-piperidinyl]ethyl]-2,3-dihydro-7-methyl-5H-thiazolo[3,2-a]pyrimidin-5-one were added portionwise while stirring at room temperature. Upon complete addition, stirring was continued for 1 hour at room temperature. After the dropwise addition of 2.6 parts of 1-chloromethyl-4-methoxybenzene, the reaction mixture was stirred overnight at room temperature. The whole was poured into water and the product was extracted with dichloromethane. The extract was dried, filtered and evaporated. The residue was purified by column chromatography (silica gel ; $CHCl_3/CH_3OH$ 95:5). The eluent of the desired fraction was evaporated and the residue was crystallized from 2-propanol. The product was filtered off and dried, yielding 2.4 parts (30.0%) of 6-[2-[4-[6-fluoro-1-[(4-methoxyphenyl)methyl]- 1H-indazol-3-yl]-1-piperidinyl]-ethyl]-2,3-dihydro-7-methyl-5H-thiazolo[3,2-a]pyrimidin-5-one; mp. 145.8 °C (compound 30).

Example 6

A mixture of 3.2 parts of 3-[2-[4-(6-fluoro-1H-indazol-3-yl)-1-piperidinyl]ethyl]-2,9-dimethyl-4H-pyrido[1,2-a]-pyrimdin-4-one, 27 parts of acetic anhydride and 15.7 parts of acetic acid was stirred for 2 hours at reflux temperature. The reaction mixture was evaporated and the residue was poured into 100 parts of water. After basifying with $NH_4OH$, the product was extracted with dichloromethane. The extract was dried, filtered and evaporated and the residue was crystallized from 2-propanol, yielding 3.3 parts (95.3%) of 3-[2-[4-(1-acetyl-6-fluoro-1H-indazol-3-yl)-1-piperidinyl]ethyl]-2,9-dimethyl-4H-pyrido[1,2-a]pyrimidin-4-one; mp. 210.3 °C (compound 46).

All other compounds listed in tables 1 to 3 were obtained following methods of preparation analogous to those described in examples 2 to 6, as is indicated in the column headed by Ex. No..

Table 1

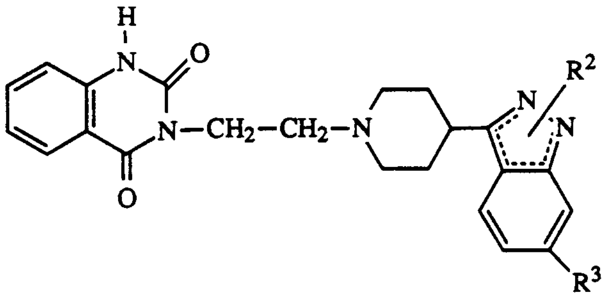

| Comp. No. | Ex. No. | $R^2$ | $R^3$ | Physical Data |
|---|---|---|---|---|
| 3 | 3 | 4-F-$C_6H_4$ | F | HCl / mp. 291.5°C |
| 4 | 3 | H | F | mp. 278.9°C |
| 5 | 3 | $CH_2$-$C_6H_5$ | F | mp. 223.5°C |
| 53 | 3 | H | $OCH_3$ | 2 HCl/$H_2O$/mp. 244.3°C |

Table 2

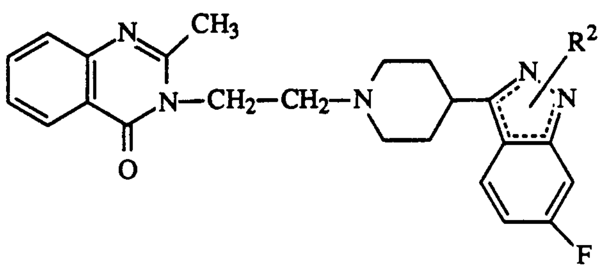

| Comp. No. | Ex. No. | $R^2$ | Physical Data |
|---|---|---|---|
| 6 | 4 | H | mp. 259.7°C |
| 7 | 3 | $CH_3$ | mp. 143.8°C |
| 8 | 3 | $CH_2$-$C_6H_5$ | mp. 98.3°C |

Table 3

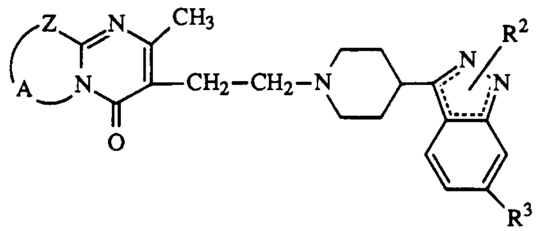

| Comp. No. | Ex. No. | -Z-A- | $R^2$ | $R^3$ | Physical Data |
|---|---|---|---|---|---|
| 9 | 3 | -CH=CH-CH=CH- | 4-F-$C_6H_4$ | F | mp. 147.5°C |
| 10 | 3 | -CH=CH-CH=CH- | H | F | mp. 259.5°C |
| 11 | 3 | -CH=CH-CH=CH- | $CH_3$ | F | mp. 149.8°C |
| 12 | 3 | -CH=CH-CH=CH- | $CH_2$-$C_6H_5$ | F | mp. 131.4°C |
| 13 | 3 | -$(CH_2)_4$- | H | F | mp. 236.2°C |
| 14 | 3 | -$(CH_2)_4$- | $CH_3$ | F | mp. 168.1°C |
| 15 | 3 | -$(CH_2)_4$- | $CH_2$-$C_6H_5$ | F | mp. 162.1°C |
| 16 | 3 | -S-CH=CH- | 4-F-$C_6H_4$ | F | (E)-2-butenedioate/ mp. 232.4°C |
| 17 | 3 | -S-CH=CH- | H | F | 1/2 $H_2O$/mp. 190.1°C |
| 18 | 3 | -S-CH=CH- | $CH_3$ | F | mp. 139.9°C |
| 19 | 3 | -S-CH=CH- | $CH_2$-$C_6H_5$ | F | mp. 127.6°C |
| 20 | 4 | -S-CH=C($CH_3$)- | 4-F-$C_6H_4$ | F | 1/2 (E)-2-butenedioate/ $H_2O$ / mp. 165.0°C |
| 21 | 3 | -S-CH=C($CH_3$)- | $CH_3$ | F | mp. 170.0°C |
| 22 | 4 | -S-CH=C($CH_3$)- | H | F | mp. 232.4°C |
| 23 | 3 | -S-CH=C($CH_3$)- | $CH_2$-$C_6H_5$ | F | mp. 141.1°C |
| 24 | 3 | -S-CH=C($CH_3$)- | 2-$CH_2$-$C_6H_5$ | F | mp. 180.0°C |
| 25 | 3 | -S-$(CH_2)_2$- | $CH_3$ | F | mp. 157.6°C |
| 26 | 3 | -S-$(CH_2)_2$- | $CH_2$-$C_6H_5$ | F | mp. 150.2°C |
| 27 | 3 | -S-$(CH_2)_2$- | 2-$CH_2$-$C_6H_5$ | F | mp. 175.2°C |
| 28 | 3 | -C($CH_3$)=CH-CH=CH- | H | F | mp. 213.1°C |
| 29 | 3 | -C($CH_3$)=CH-CH=CH- | $CH_2C_6H_5$ | F | mp. 139°C |
| 30 | 5 | -S-$(CH_2)_2$- | $CH_2$-(4-$OCH_3C_6H_4$) | F | mp. 145.8°C |
| 31 | 3 | -CH=C($CH_3$)-O- | $CH_2$-$C_6H_5$ | F | mp. 136.1°C |
| 32 | 4 | -S-CH=C($CH_3$)- | $CH_2$-(4-F-$C_6H_4$) | F | mp. 139.2°C |

| Comp. No. | Ex. No. | -Z-A- | $R^2$ | $R^3$ | Physical Data |
|---|---|---|---|---|---|
| 33 | 3 | $-C(CH_3)=CH-CH=CH-$ | H | $OCH_3$ | mp. 210.0°C |
| 34 | 3 | -S-CH=CH- | H | $OCH_3$ | mp. 183.2°C |
| 35 | 3 | -S-CH=CH- | H | H | 2 HCl/mp. 196.4°C |
| 36 | 5 | $-(CH_2)_4-$ | 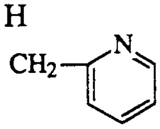 | F | (E)-2-butenedioate(1:1) mp. 151.2°C |
| 37 | 3 | $-C(CH_3)=CH-CH=CH-$ | $-CH_2CH_2OH$ | F | mp. 176.0°C |
| 38 | 3 | $-(CH_2)_4-$ | $-CH_2CH_2OH$ | F | mp. 140.1°C |
| 39 | 3 | $-S-(CH_2)_2-$ | $-CH_2CH_2OH$ | F | mp. 165.6°C |
| 40 | 3 | $-C(CH_3)=CH-CH=CH-$ | H | H | mp. 180.2°C |
| 41 | 3 | $-CH=C(CH_3)-O-$ | $CH_3$ | F | mp. 157.7°C |
| 42 | 3 | $-(CH_2)_4-$ | H | H | mp. 188.1°C |
| 43 | 3 | -S-CH=CH- | $-CH_2CH_2OH$ | F | mp. 197.2°C |
| 44 | 5 | $-(CH_2)_4-$ | 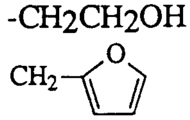 | F | mp. 179.4°C |
| 45 | 3 | $-S-CH_2-CH_2$ | H | H | mp. 210.3°C |
| 46 | 6 | $-C(CH_3)=CH-CH=CH-$ | $COCH_3$ | F | |
| 47 | 6 | $-C(CH_3)=CH-CH=CH-$ | $CO(4-Cl-C_6H_5)$ | F | mp. 181.6°C |
| 48 | 6 | $-C(CH_3)=CH-CH=CH-$ | $COOC_2H_5$ | F | mp. 183.1°C |
| 49 | 3 | $-S-CH_2-CH_2-$ | H | H | |
| 50 | 6 | $-C(CH_3)=CH-CH=CH-$ | $COCH_3$ | H | mp. 160.7°C |
| 51 | 6 | $-(CH_2)_4-$ | $COCH_3$ | H | mp. 149.3°C |
| 52 | 3 | $-S-CH_2-CH_2-$ | $COCH_3$ | H | |

C) Pharmacological examples

The activity of the subject compounds as antagonists of neurotransmitters is evidenced by the experimental data obtained in at least one of two different test procedures, viz., the combined apomorphine-, tryptamine- and norepinephrine tests in rats and the apomorphine test in dogs. The tests are carried out following the procedures described hereafter and the experimental data are summarized in table 4. The antihypertensive effect of the instant compounds is evidenced by the "Blood pressure lowering effect in spontaneous hypertensive rats" test. The experimental data are summarized in table 5.

Example 7

The combined apomorphine (APO)-, tryptamine (TRY)- and norepinephrine (NOR) test in rats.

The experimental animals used in this test were adult male Wistar rats (weight 240 ± 10g). After an overnight fast, the animals were treated subcutaneously or orally with an aqueous solution of the compound under investigation (1ml/100g body weight) (time = zero) and put in isolated observation cages. Thirty minutes thereafter (time = 30 minutes) 1.25 mg/kg of apomorphine hydrochloride (APO) was injected intravenously and the rats were observed over a 1 hour period for the presence or absence of the following apomorphine-induced phenomena: agitation and stereotypic chewing. At the end of this 1 hour period (time = 90 minutes) the same animals were injected intravenously with 40 mg/kg of tryptamine (TRY) and the presence of the typical tryptamine-induced bilateral tonic seizures and hyperaemia of the ears was noted. Two hours after pretreatment (time = 120 minutes) finally, the same animals were challenged with 1.25 mg/kg intravenously of norephinephrine (NOR) and possible mortality was looked for up to 60 minutes later.

The table 4 gives the $ED_{50}$-values of a number of the compounds under consideration. As used herein, the $ED_{50}$-value represents the dose which protects 50% of the animals from apomorphine-, tryptamine- or norepinephrine-induced phenomena.

The apomorphine test in dogs (APO-dog).

The method used is described by P.A.J. Janssen and C.J.E. Niemegeers in Arzneim.-Forsch. (Drug Res.), 9,765-767 (1959). The compounds listed in table 4 were administered subcutaneously or orally to beagle dogs at different doses and the animals were challenged one hour thereafter with a standard dose of 0.31 mg/kg (s.c.) of apomorphine.

The table 4 gives the $ED_{50}$-values of a number of the compounds under consideration. As used herein, the $ED_{50}$ value represents the dose which protects 50% of the animals from emesis.

The compounds listed in table 4 are not given for the purpose of limiting the invention thereto but only to exemplify the useful pharmacological activities of all the compounds within the scope of formula (I).

Table 4

| Comp. No. | Combined test in rats; $ED_{50}$ in mg/kg | | | | (APO)-dog test, $ED_{50}$ in mg/kg |
|---|---|---|---|---|---|
| | (APO) | (TRY)-convulsions | (TRY)-hyperaemia | (NOR) | |
| 2 | 5 | 5 | 0.005 | 0.31 | 0.002 |
| 9 | >10 | 5 | 0.08 | 5 | |
| 11 | 5 | 0.31 | 0.005 | 0.08 | 0.12 |
| 13 | >10 | 1.25 | 0.02 | 0.31 | 0.015 |
| 16 | >10 | 5 | 0.08 | >10 | |
| 17 | 5 | 5 | 0.03 | 2.0 | 0.004 |
| 18 | 5 | 1.25 | 0.005 | 1.25 | 0.2 |
| 20 | >10 | ≧10 | 0.08 | >10 | |
| 21 | 1.25 | 0.08 | 0.005 | 0.31 | 0.12 |
| 25 | >10 | 1.25 | 0.01 | 0.31 | <0.015 |
| 29 | 1.25 | 5 | ≦0.16 | 1.25 | 0.004 |
| 30 | >10 | 10 | 0.08 | 5 | >0.63 |
| 31 | >10 | 1.25 | 0.02 | 0.31 | >0.63 |
| 34 | >10 | >10 | 1.25 | 1.25 | >0.01 |

Example 8

Blood pressure lowering effect in spontaneous hypertensive rats (SHR).

Adult spontaneous hypertensive rats (6 months of age) were anesthetized by ether inhalation. The femoral artery was dissected and cannulated, and the catheter was connected to a strain-gauge blood pressure transducer. When the animals were fully awake, they were restrained and the systolic and diastolic

arterial blood pressure were continuously recorded. An observation period of at least 30 min. preceded the administration of the test compound. All test compounds were dissolved in 20% polypropylene glycol and injected intraperitoneally. After administration of the test drug the systolic and diastolic arterial blood pressure and the heart rate were recorded during a period of 120 minutes. The average blood pressure and heart rate was calculated from the results obtained at various time intervals after administration of the test drug. The following table illustrates the lowering of the systolic and diastolic blood pressure.

Table 5

| Comp. No. | SBP (mm Hg) | DBP (mm Hg) |
|---|---|---|
| 2 | -140 | -100 |
| 9 | -40 | -30 |
| 11 | -130 | -90 |
| 13 | -120 | -80 |
| 18 | -90 | -70 |
| 25 | -170 | -90 |
| 28 | -140 | -100 |
| 29 | -30 | -30 |
| 30 | -70 | -55 |
| 31 | -80 | -60 |
| 34 | -20 | -10 |
| 46 | -100 | -65 |
| 47 | -90 | -60 |
| 48 | -110 | -85 |
| 53 | -60 | -45 |

D) Composition Examples

Example 9 : ORAL DROPS

500 Grams of the A.I. was dissolved in 0.51 of 2-hydroxypropanoic acid and 1.5 l of the polyethylene glycol at 60~80 °C. After cooling to 30~40 °C there were added 35 l of polyethylene glycol and the mixture was stirred well. Then there was added a solution of 1750 grains of sodium saccharin in 2.5 l of purified water and while stirring there were added 2.5 l of cocoa flavor and polyethylene glycol q.s. to a volume of 50 l, providing an oral drop solution comprising 10 mg/ml of A.I.. The resulting solution was filled into suitable containers.

Example 10 : ORAL SOLUTION

9 Grams of methyl 4-hydroxybenzoate and 1 gram of propyl 4-hydroxybenzoate were dissolved in 4 l of boiling purified water. In 3 l of this solution were dissolved first 10 grams of 2,3-dihydroxybutanedioic acid and thereafter 20 grams of the A.I. The latter solution was combined with the remaining part of the former solution and 12 l 1,2,3-propanetriol and 3 l of sorbitol 70% solution were added thereto. 40 Grams of sodium saccharin were dissolved in 0.5 l of water and 2 ml of raspberry and 2 ml of gooseberry essence were added. The latter solution was combined with the former, water was added q.s. to a volume of 20 l providing an oral solution comprising 5 mg of the active ingredient per teaspoonful (5 ml). The resulting solution was filled in suitable containers.

Example 11 : CAPSULES

20 Grams of the A.I., 6 grams sodium lauryl sulfate, 56 grams starch, 56 grams lactose, 0.8 grams colloidal silicon dioxide, and 1.2 grams magnesium stearate were vigorously stirred together. The resulting mixture was subsequently filled into 1000 suitable hardened gelatin capsules, comprising each 20 mg of the active ingredient.

Example 12 : FILM-COATED TABLETS

Preparation of tablet core

A mixture of 100 grams of the A.I., 570 grams lactose and 200 grams starch was mixed well and thereafter humidified with a solution of 5 grams sodium dodecyl sulfate and 10 grams polyvinylpyrrolidone (Kollidon-K 90 ®) in about 200 ml of water. The wet powder mixture was sieved, dried and sieved again. Then there was added 100 grams microcrystalline cellulose (Avicel ®) and 15 grams hydrogenated vegetable oil (Sterotex ®). The whole was mixed well and compressed into tablets, giving 10.000 tablets, each containing 10 mg of the active ingredient.

Coating

To a solution of 10 grams methyl cellulose (Methocel 60 HG®) in 75 ml of denaturated ethanol there was added a solution of 5 grams of ethyl cellulose (Ethocel 22 cps ®) in 150 ml of dichloromethane. Then there were added 75 ml of dichloromethane and 2.5 ml 1,2,3-propanetriol. 10 Grams of polyethylene glycol was molten and dissolved in 75 ml of dichloromethane. The latter solution was added to the former and then there were added 2.5 grams of magnesium octadecanoate, 5 grams of polyvinylpyrrolidone and 30 ml of concentrated colour suspension (Opaspray K-1-2109®) and the whole was homogenated. The tablet cores were coated with the thus obtained mixture in a coating apparatus.

Example 13 : INJECTABLE SOLUTION

1.8 Grams methyl 4-hydroxybenzoate and 0.2 grams propyl 4-hydroxybenzoate were dissolved in about 0.5 l of boiling water for injection. After cooling to about 50 °C there were added while stirring 4 grams lactic acid, 0.05 grams propylene glycol and 4 grams of the A.I.. The solution was cooled to room temperature and supplemented with water for injection q.s. ad 1 l, giving a solution comprising 4 mg/ml of A.I.. The solution was sterilized by filtration (U.S.P. XVII p. 811) and filled in sterile containers.

Example 14 : SUPPOSITORIES

3 Grams A.I. was dissolved in a solution of 3 grams 2,3-dihydroxybutanedioic acid in 25 ml polyethylene glycol 400.12 Grams surfactant (SPAN®) and triglycerides (Witepsol 555 ®) q.s. ad 300 grams were molten together. The latter mixture was mixed well with the former solution. The thus obtained mixture was poured into moulds at a temperature of 37-38 °C to form 100 suppositories each containing 30 mg/ml of the A.I..

**Claims**

**Claims for the following Contracting States : AT, BE, CH, DE, FR, GB, IT, LI, LU, NL, SE**

**1.** A chemical compound having the formula

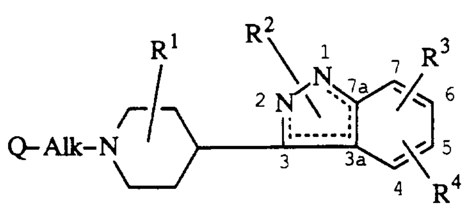

(I),

a pharmaceutically acceptable acid addition salt or a stereochemically isomeric form thereof, wherein

$R^1$ is hydrogen or $C_{1-6}$alkyl;

$R^2$ is hydrogen; $C_{1-6}$alkyl: hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl;

$R^3$ and $R^4$ each independently are hydrogen, halo, hydroxy, $C_{1-6}$alkyloxy or $C_{1-6}$alkyl;
Alk is $C_{1-4}$alkanediyl; and
Q is a bicyclic heterocyclic radical of formula

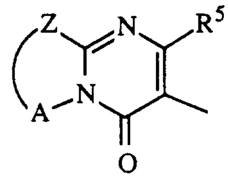

(a),

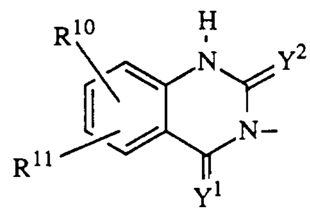

(b) or

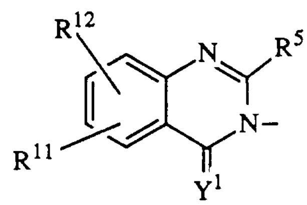

(c);

wherein
$R^5$ is hydrogen or $C_{1-6}$alkyl;
Z is -S- or -$CR^6$=$CR^7$-; said $R^6$ and $R^7$ each independently being hydrogen or $C_{1-6}$alkyl; or Z is -$CH_2$- wherein one hydrogen atom may be replaced by hydroxy or $C_{1-6}$alkyl;
A is a bivalent radical -$CH_2$-$CH_2$- or -$CH_2$-$CH_2$-$CH_2$- wherein in the latter two radicals one or two hydrogen atoms may be replaced by $C_{1-6}$alkyl; or A is a bivalent radical -$CR^8$=$CR^9$-, wherein $R^8$ and $R^9$ each independently are hydrogen, halo, amino or $C_{1-6}$alkyl; or when Z is -S-, then A may also be -$CR^{10}$=N-, $R^{10}$ being hydrogen or $C_{1-6}$alkyl; or when Z is -$CR^6$=$CR^7$-, then A also may be -O- ; and
$Y^1$ and $Y^2$ each independently are O or S;
$R^{11}$ is hydrogen, halo, $C_{1-6}$alkyl, $C_{1-6}$alkyloxy, trifluoromethyl, nitro, mono- or di($C_{1-6}$alkyl)amino, $C_{1-10}$alkylcarbonylamino, cyano, $C_{1-10}$alkylcarbonyloxy, phenylmethoxy or azido;
$R^{12}$ is hydrogen or halo; and
aryl is phenyl optionally substituted with up to three substituents independently selected from $C_{1-6}$alkyl, $C_{1-6}$alkyloxy, hydroxy, halo, amino, nitro and trifluoromethyl; pyridinyl, furanyl or $C_{1-6}$alkyl substituted furanyl.

**2.** A chemical compound according to claim 1 wherein $R^1$ is hydrogen; $R^2$ is substituted on $N^1$, $R^3$ and $R^4$ each independently are hydrogen, $C_{1-6}$alkyloxy or halo; Q is a radical of formula (a) wherein $R^5$ is $C_{1-6}$alkyl.

**3.** A chemical compound according to claim 1 wherein $R^2$ is hydrogen, $C_{1-6}$alkyl, phenyl optionally sbustituted with halo or trifluoromethyl or phenylmethyl optionally substituted with halo, $C_{1-6}$alkyloxy or trifluoromethyl; $R^3$ is halo; $R^4$ is hydrogen.

**4.** A chemical compound according to claim 1 wherein $R^3$ is 6-fluoro.

**5.** A pharmaceutical composition comprising a suitable pharmaceutical carrier and as active ingredient a therapeutically effective amount of a compound as claimed in any one of claims 1 to 4.

**6.** An antihypertensive pharmaceutical composition comprising a suitable pharmaceutical carrier and as active ingredient an effective antihypertensive amount of a compound as claimed in any one of claims 1 to 4.

**7.** A method of preparing a pharmaceutical composition as claimed in claims 5 or 6, characterized in that a therapeutically effective amount of a compound as defined in any of claims 1 to 4 is intimately mixed with suitable pharmaceutical carriers.

**8.** A compound as claimed in any one of claims 1 to 4 for use as a medicine.

**9.** A compound as claimed in any one of claims 1 to 4 for use as an antihypertensive medicine.

**10.** A process for preparing a chemical compound as claimed in any one of claims 1 to 4, characterized by
a) N-alkylating a piperidine of formula

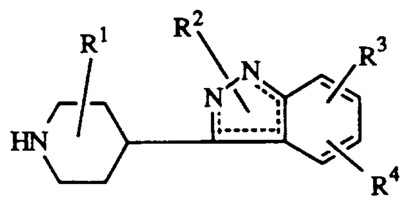

(III)

wherein $R^1$, $R^2$, $R^3$ and $R^4$ are as defined under formula (I) with an alkylating reagent Q-Alk-W (II) wherein Q and Alk are as defined under formula (I) and wherein W is a leaving group, in a reaction-inert solvent, optionally in the presence of a base and optionally in the presence of an alkali metal iodide, by stirring at an elevated temperature, or
b) N-alkylating or N-acylating a compound of formula

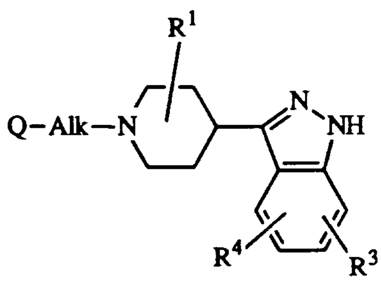

(I-b),

wherein Q, Alk, $R^1$, $R^3$ and $R^4$ are as defined under formula (I), with an alkylating or acylating reagent of formula $R^{2-a}W^1$ (IV) wherein $W^1$ is a leaving group and $R^{2-a}$ is $C_{1-6}$alkyl; hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl, by treatment with a base in a reaction-inert solvent, optionally at an elevated temperature, thus yielding a compound of formula

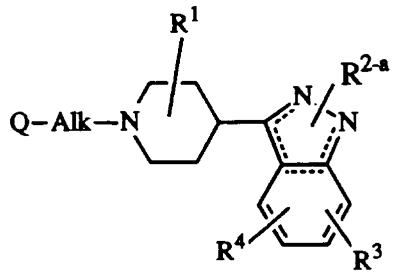

(I-a),

wherein Q, Alk, $R^1$, $R^3$ and $R^4$ are as defined under formula (I) and $R^{2-a}$ is $C_{1-6}$alkyl; hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl;

c) cyclizing a intermediate of formula

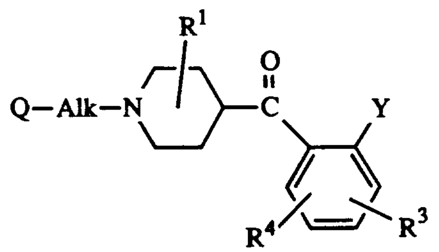

(V),

wherein Q, Alk, $R^1$, $R^3$ and $R^4$ are as defined under formula (I) and Y is a leaving group with a hydrazine derivative $R^2$-NH-$NH_2$ (VI) or an acid addition salt thereof, in a reaction-inert solvent in the presence of a base, optionally at a enhanced temperature; and if desired, converting the compounds of formula (I) into a therapeutically active non-toxic acid addition salt form by treatment with a acid; or conversely, converting the acid salt into the free base with alkali; and/or preparing stereochemically isomeric forms thereof.

**Claim for the following Contracting States : ES, GR.**

**1.** A process for preparing a chemical compound having the formula

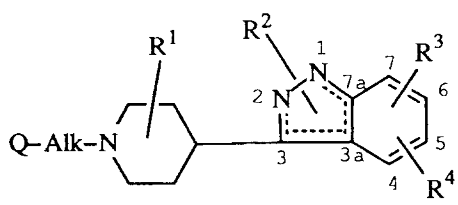

(I),

a pharmaceutically acceptable acid addition salt or a stereochemically isomeric form thereof, wherein

$R^1$ is hydrogen or $C_{1-6}$alkyl;

$R^2$ is hydrogen; $C_{1-6}$alkyl; hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl;

$R^3$ and $R^4$ each independently are hydrogen, halo, hydroxy, $C_{1-6}$alkyloxy or $C_{1-6}$alkyl;

Alk is $C_{1-4}$alkanediyl; and
Q is a bicyclic heterocyclic radical of formula

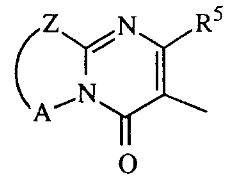

(a),

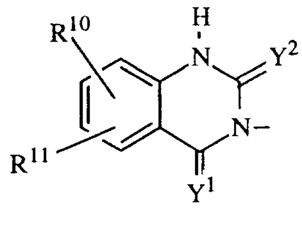

(b) or

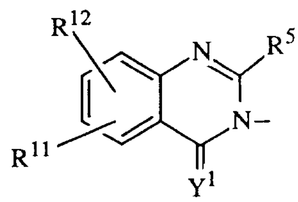

(c);

wherein
$R^5$ is hydrogen or $C_{1-6}$alkyl;
Z is -S- or $-CR^6=CR^7-$; said $R^6$ and $R^7$ each independently being hydrogen or $C_{1-6}$alkyl; or Z is $-CH_2-$ wherein one hydrogen atom may be replaced by hydroxy or $C_{1-6}$alkyl;
A is a bivalent radical $-CH_2-CH_2-$ or $-CH_2-CH_2-CH_2-$ wherein in the latter two radicals one or two hydrogen atoms may be replaced by $C_{1-6}$alkyl; or A is a bivalent radical $-CR^8=CR^9-$, wherein $R^8$ and $R^9$ each independently are hydrogen, halo, amino or $C_{1-6}$alkyl; or when Z is -S-, then A may also be $-CR^{10}=N-$, $R^{10}$ being hydrogen or $C_{1-6}$alkyl; or when Z is $-CR^6=CR^7-$, then A also may be -O- ; and
$Y^1$ and $Y^2$ each independently are O or S;
$R^{11}$ is hydrogen, halo, $C_{1-6}$alkyl, $C_{1-6}$alkyloxy, trifluoromethyl, nitro, amino, mono- or di($C_{1-6}$alkyl)-amino, $C_{1-10}$alkylcarbonylamino, cyano, hydroxy, $C_{1-10}$alkylcarbonyloxy, phenylmethoxy or azido;
$R^{12}$ is hydrogen or halo; and
aryl is phenyl optionally substituted with up to three substituents independently selected from $C_{1-6}$alkyl, $C_{1-6}$alkyloxy, hydroxy, halo, amino, nitro and trifluoromethyl; pyridinyl, furanyl or $C_{1-6}$alkyl substituted furanyl, characterized by
a) N-alkylating a piperidine of formula

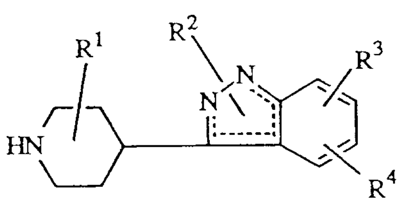

(III)

wherein $R^1$, $R^2$, $R^3$ and $R^4$ are as defined under formula (I) with an alkylating reagent Q-Alk-W (II) wherein Q and Alk are as defined under formula (I) and wherein W is a leaving group, in a reaction-inert solvent, optionally in the presence of a base and optionally in the presence of an alkali metal iodide, by stirring at an elevated temperature, or

b) N-alkylating or N-acylating a compound of formula

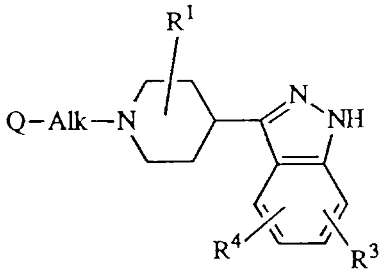

(I-b),

wherein Q, Alk, $R^1$, $R^3$ and $R^4$ are as defined under formula (I), with an alkylating or acylating reagent of formula $R^{2-a}W^1$ (IV) wherein $W^1$ is a leaving group and $R^{2-a}$ is $C_{1-6}$alkyl; hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl, by treatment with a base in a reaction-inert solvent, optionally at an elevated temperature, thus yielding a compound of formula

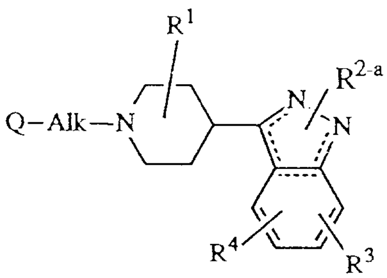

(I-a),

wherein Q, Alk, $R^1$, $R^3$ and $R^4$ are as defined under formula (I) and $R^{2-a}$ is $C_{1-6}$alkyl; hydroxy$C_{1-6}$alkyl; phenyl optionally substituted with up to three substituents independently selected from $C_{1-6}$alkyl, $C_{1-6}$alkyloxy, hydroxy, halo, amino, nitro and trifluoromethyl; aryl$C_{1-6}$alkyl; $C_{1-6}$alkylcarbonyl; $C_{1-6}$alkyloxycarbonyl or phenylcarbonyl, wherein phenyl is optionally substituted with up to three substituents independently selected from $C_{1-6}$alkyl, $C_{1-6}$alkyloxy, hydroxy, halo, amino, nitro and trifluoromethyl;

c) cyclizing an intermediate of formula

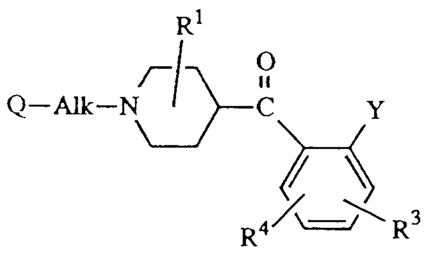

(V),

wherein Q, Alk, $R^1$, $R^3$ and $R^4$ are as defined under formula (I) and Y is a leaving group with a hydrazine derivative $R^2$-NH-$NH_2$ (VI) or an acid addition salt thereof, in a reaction-inert solvent in the presence of a base, optionally at an enhanced temperature; and if desired, converting the compounds of formula (I) into a therapeutically active non-toxic acid addition salt form by treatment with an acid; or conversely, converting the acid salt into the free base with alkali; and/or preparing stereochemically isomeric forms thereof.

**Patentansprüche**

**Patentansprüche für folgende Vertragsstaaten : AT, BE, CH, DE, FR, GB, IT, LI, LU, NL, SE**

1. Chemische Verbindung mit der Formel

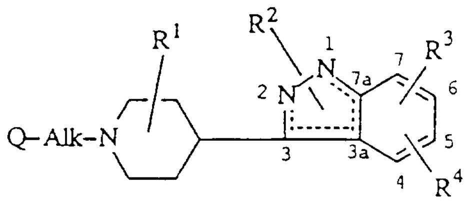

(I),

ein pharmazeutisch annehmbares Säureadditionssalz oder eine stereochemisch isomere Form hievon, worin

$R^1$ Wasserstoff oder $C_{1-6}$-Alkyl bedeutet;

$R^2$ für Wasserstoff; $C_{1-6}$-Alkyl; Hydroxy-$C_{1-6}$-alkyl, gegebenenfalls durch bis zu drei Substituenten, die unabhängig voneinander unter $C_{1-6}$-Alkyl, $C_{1-6}$-Alkyloxy, Hydroxy, Halogen, Amino, Nitro und Trifluormethyl ausgewählt sind, substituiertes Phenyl; Aryl-$C_{1-6}$-alkyl; $C_{1-6}$-Alkylcarbonyl; $C_{1-6}$-Alkyloxycarbonyl oder Phenylcarbonyl steht, worin das Phenyl gegebenenfalls durch bis zu drei Substituenten, unabhängig voneinander ausgewählt unter $C_{1-6}$-Alkyl, $C_{1-6}$-Alkyloxy, Hydroxy, Halogen, Amino, Nitro und Trifluormethyl, substituiert ist;

$R^3$ und $R^4$ jeweils unabhängig voneinander Wasserstoff, Halogen, Hydroxy, $C_{1-6}$-Alkyloxy oder $C_{1-6}$-Alkyl darstellen;

Alk für $C_{1-4}$-Alkandiyl steht; und

Q einen bicyclischen heterocyclischen Rest der Formel

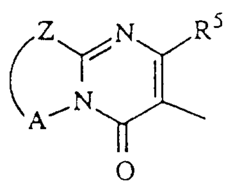

(a),

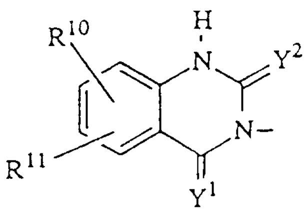

(b) oder

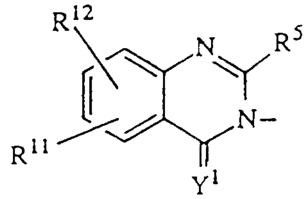

(c)

bedeutet, worin

$R^5$ Wasserstoff oder $C_{1-6}$-Alkyl darstellt;

Z für -S- oder $-CR^6=CR^7-$ steht; worin die Reste $R^6$ und $R^7$ jeweils unabhängig voneinander Wasserstoff oder $C_{1-6}$-Alkyl bedeuten; oder Z für $-CH_2-$ steht, worin ein Wasserstoffatom durch Hydroxy oder $C_{1-6}$-Alkyl ersetzt sein kann;

A einen zweiwertigen Rest $-CH_2-CH_2-$ oder $-CH_2-CH_2-CH_2-$ bedeutet, wobei in den letztgenannten beiden Resten ein oder zwei Wasserstoffatome durch $C_{1-6}$-Alkyl ersetzt sein können; oder A einen zweiwertigen Rest $-CR^8=CR^9-$ darstellt, worin $R^8$ und $R^9$ jeweils unabhängig voneinander Wasserstoff, Halogen, Amino oder $C_{1-6}$-Alkyl bedeuten; oder, wenn Z für -S- steht, A auch $-CR^{10}=N-$ darstellen kann, worin $R^{10}$ Wasserstoff oder $C_{1-6}$-Alkyl bedeutet; oder, wenn Z für $-CR^6=CR^7-$ steht, A auch -O- sein kann; und

$Y^1$ und $Y^2$ jeweils unabhängig O oder S darstellen;

$R^{11}$ für Wasserstoff, Halogen, $C_{1-6}$-Alkyl, $C_{1-6}$-Alkyloxy, Trifluormethyl, Nitro, Mono- oder Di($C_{1-6}$-alkyl)amino, $C_{1-10}$-Alkylcarbonylamino, Cyano, $C_{1-10}$-Alkylcarbonyloxy, Phenylmethoxy oder Azido steht;

$R^{12}$ Wasserstoff oder Halogen bedeutet; und

Aryl für Phenyl, das gegebenenfalls durch bis zu drei Substituenten, die unabhängig voneinander unter $C_{1-6}$-Alkyl, $C_{1-6}$-Alkyloxy, Hydroxy, Halogen, Amino, Nitro und Trifluormethyl ausgewählt sind, subscituiert ist; Pyridinyl, Furanyl oder $C_{1-6}$-Alkyl-substituiertes Furanyl steht.

**2.** Chemische Verbindung nach Anspruch 1, worin $R^1$ Wasserstoff darstellt; $R^2$ als Substituent am $N^1$ vorliegt, $R^3$ und $R^4$ jeweils unabhängig Wasserstoff, $C_{1-6}$-Alkyloxy oder Halogen bedeuten; Q einen Rest der Formel (a) bedeutet, worin $R^5$ für $C_{1-6}$-Alkyl steht.

**3.** Chemische Verbindung nach Anspruch 1, worin $R^2$ Wasserstoff, $C_{1-6}$-Alkyl, gegebenenfalls durch Halogen oder Trifluormethyl substituiertes Phenyl oder gegebenenfalls durch Halogen, $C_{1-6}$-Alkyloxy oder Trifluormethyl substituiertes Phenylmethyl darstellt; $R^3$ Halogen bedeutet; $R^4$ Wasserstoff darstellt.

**4.** Chemische Verbindung nach Anspruch 1, worin $R^3$ für 6-Fluor steht.

**5.** Pharmazeutische Zusammensetzung, umfassend einen geeigneten pharmazeutischen Träger und als wirksamen Bestandteil eine therapeutisch wirksame Menge einer Verbindung, wie in einem der Ansprüche 1 bis 4 beansprucht.

**6.** Antihypertensive pharmazeutische Zusammensetzung, umfassend einen geeigneten pharmazeutischen Träger und als wirksamen Bestandteil eine wirksame antihypertensive Menge einer Verbindung, wie in einem der Ansprüche 1 bis 4 beansprucht.

**7.** Verfahren zur Herstellung einer pharmazeutischen Zusammensetzung, wie in den Ansprüchen 5 oder 6 beansprucht, dadurch gekennzeichnet, daß eine therapeutisch wirksame Menge einer Verbindung, wie in einem der Ansprüche 1 bis 4 definiert, innig mit geeigneten pharmazeutischen Trägern vermischt wird.

**8.** Verbindung nach einem der Ansprüche 1 bis 4 zur Anwendung als ein Medikament.

**9.** Verbindung nach einem der Ansprüche 1 bis 4 zur Anwendung als ein antihypertensives Medikament.

**10.** Verfahren zur Herstellung einer chemischen Verbindung nach einem der Ansprüche 1 bis 4, gekennzeichnet durch

a) N-Alkylieren eines Piperidins der Formel

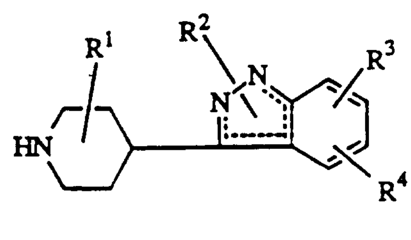

(III) ,

worin $R^1$, $R^2$, $R^3$ und $R^4$ wie unter Formel (I) definiert sind, mit einem Alkylierungsmittel Q-Alk-W (II), worin Q und Alk wie unter Formel (I) definiert sind und worin W eine Leaving-Gruppe bezeichnet, in einem reaktionsinerten Lösungsmittel, gewünschtenfalls in Anwesenheit einer Base und gewünschtenfalls in Anwesenheit eines Alkalimetalliodids, durch Rühren bei erhöhter Temperatur, oder

b) N-Alkylieren oder N-Acylieren einer Verbindung der Formel

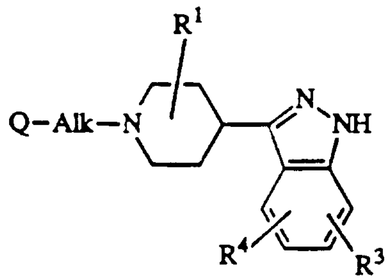

(I-b),

worin Q, Alk, $R^1$, $R^3$ und $R^4$ wie unter Formel (I) definiert sind, mit einem Alkylierungs- oder Acylierungsmittel der Formel $R^{2-a}W^1$ (IV), worin $W^1$ eine Leaving-Gruppe darstellt und $R^{2-a}$ für $C_{1-6}$-Alkyl; Hydroxy-$C_{1-6}$-alkyl; Phenyl, das gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist; Aryl-$C_{1-6}$-alkyl; $C_{1-6}$-Alkylcarbonyl; $C_{1-6}$-Alkyloxycarbonyl oder Phenylcarbonyl steht, worin das Phenyl gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist, durch Behandlung mit einer Base in einem reaktionsinerten Lösungsmittel, gegebenenfalls bei erhöhter Temperatur, unter Ausbildung einer Verbindung der Formel

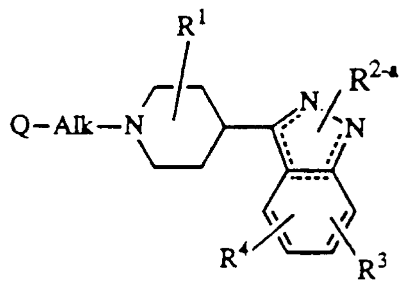

(I-a),

worin Q, Alk, $R^1$, $R^3$ und $R^4$ wie unter Formel (I) definiert sind und $R^{2-a}$ für $C_{1-6}$-Alkyl; Hydroxy-$C_{1-6}$-alkyl; Phenyl, das gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist; Aryl-$C_{1-6}$-alkyl; $C_{1-6}$-Alkylcarbonyl; $C_{1-6}$-Alkyloxycarbonyl oder Phenylcarbonyl steht, worin das Phenyl gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist;

c) Cyclisieren eines Zwischenprodukts der Formel

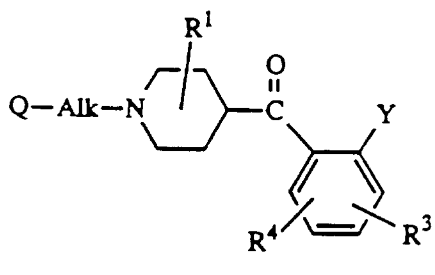

(V),

worin Q, Alk, $R^1$, $R^3$ und $R^4$ wie unter Formel (I) definiert sind und Y eine Leaving-Gruppe bezeichnet, mit einem Hydrazinderivat $R^2$-NH-$NH_2$ (VI) oder einem Säureadditionssalz hievon in einem reaktionsinerten Lösungsmittel in Anwesenheit einer Base, gewünschtenfalls bei erhöhter Temperatur;

und gewünschtenfalls Überführen der Verbindungen der Formel (I) in eine therapeutisch wirksame nicht-toxische Säureadditionssalzform durch Behandlung mit einer Säure; oder umgekehrt Überführen

des Säuresalzes in die freie Base mit Alkali; und/oder Bereiten stereochemisch isomerer Formen hievon.

**Patentanspruch für folgende Vertragsstaaten: ES, GR**

**1.** Verfahren zur Herstellung einer chemischen Verbindung mit der Formel

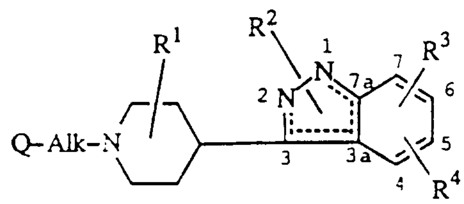

(I),

eines pharmazeutisch annehmbaren Säureadditionssalzes oder einer stereochemisch isomeren Form hievon, worin

$R^1$ Wasserstoff oder $C_{1-6}$-Alkyl bedeutet;

$R^2$ für Wasserstoff; $C_{1-6}$-Alkyl; Hydroxy-$C_{1-6}$-alkyl; gegebenenfalls durch bis zu drei Substituenten, die unabhängig voneinander unter $C_{1-6}$-Alkyl, $C_{1-6}$-Alkyloxy, Hydroxy, Halogen, Amino, Nitro und Trifluormethyl ausgewählt sind, substituiertes Phenyl; Aryl-$C_{1-6}$-alkyl; $C_{1-6}$-Alkylcarbonyl; $C_{1-6}$-Alkyloxycarbonyl oder Phenylcarbonyl steht, worin das Phenyl gegebenenfalls durch bis zu drei Substituenten, unabhängig voneinander ausgewählt unter $C_{1-6}$-Alkyl, $C_{1-6}$-Alkyloxy, Hydroxy, Halogen, Amino, Nitro und Trifluormethyl, substituiert ist;

$R^3$ und $R^4$ jeweils unabhängig voneinander Wasserstoff, Halogen, Hydroxy, $C_{1-6}$-Alkyloxy oder $C_{1-6}$-Alkyl darstellen;

Alk für $C_{1-4}$-Alkandiyl steht; und

Q einen bicyclischen heterocyclischen Rest der Formel

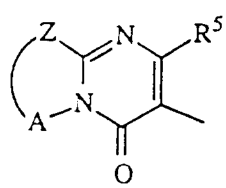

(a),

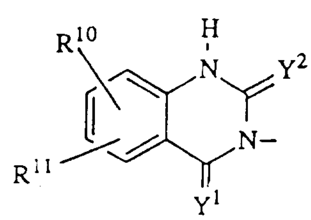

(b) oder

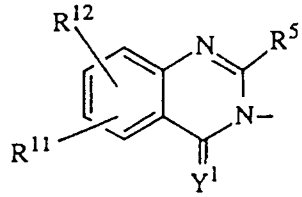

(c)

bedeutet, worin

$R^5$ Wasserstoff oder $C_{1-6}$-Alkyl darstellt;

Z für -S- oder -$CR^6$ = $CR^7$- steht; worin die Reste $R^6$ und $R^7$ jeweils unabhängig voneinander

Wasserstoff oder $C_{1-6}$-Alkyl bedeuten; oder Z für $-CH_2-$ steht, worin ein Wasserstoffatom durch Hydroxy oder $C_{1-6}$-Alkyl ersetzt sein kann;

A einen zweiwertigen Rest $-CH_2-CH_2-$ oder $-CH_2-CH_2-CH_2-$ bedeutet, wobei in den letztgenannten beiden Resten ein oder zwei Wasserstoffatome durch $C_{1-6}$-Alkyl ersetzt sein können; oder A einen zweiwertigen Rest $-CR^8=CR^9-$ darstellt, worin $R^8$ und $R^9$ jeweils unabhängig voneinander Wasserstoff, Halogen, Amino oder $C_{1-6}$-Alkyl bedeuten; oder, wenn Z für -S- steht, A auch $-CR^{10}=N-$ darstellen kann, worin $R^{10}$ Wasserstoff oder $C_{1-6}$-Alkyl bedeutet; oder, wenn Z für $-CR^6=CR^7-$ steht, A auch -O- sein kann; und

$Y^1$ und $Y^2$ jeweils unabhängig O oder S darstellen;

$R^{11}$ für Wasserstoff, Halogen, $C_{1-6}$-Alkyl, $C_{1-6}$-Alkyloxy, Trifluormethyl, Nitro, Mono- oder Di($C_{1-6}$-alkyl)amino, $C_{1-10}$-Alkylcarbonylamino, Cyano, $C_{1-10}$-Alkylcarbonyloxy, Phenylmethoxy oder Azido steht;

$R^{12}$ Wasserstoff oder Halogen bedeutet; und

Aryl für Phenyl, das gegebenenfalls durch bis zu drei Substituenten, die unabhängig voneinander unter $C_{1-6}$-Alkyl, $C_{1-6}$-Alkyloxy, Hydroxy, Halogen, Amino, Nitro und Trifluormethyl ausgewählt sind, substituiert ist; Pyridinyl, Furanyl oder $C_{1-6}$-Alkyl-substituiertes Furanyl steht, gekennzeichnet durch

a) N-Alkylieren eines Piperidins der Formel

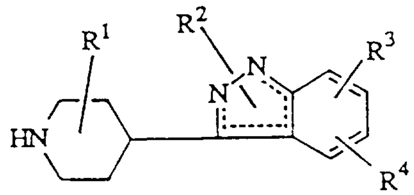

(III) .

worin $R^1$, $R^2$, $R^3$ und $R^4$ wie unter Formel (I) definiert sind, mit einem Alkylierungsmittel Q-Alk-W (II), worin Q und Alk wie unter Formel (I) definiert sind und worin W eine Leaving-Gruppe bezeichnet, in einem reaktionsinerten Lösungsmittel, gewünschtenfalls in Anwesenheit einer Base und gewünschtenfalls in Anwesenheit eines Alkalimetalliodids, durch Rühren bei erhöhter Temperatur, oder

b) N-Alkylieren oder N-Acylieren einer Verbindung der Formel

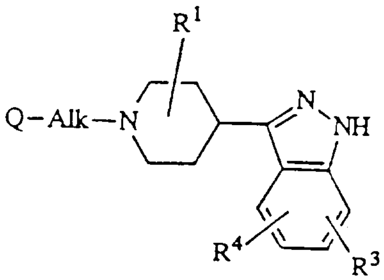

(I-b),

worin Q, Alk, $R^1$, $R^3$ und $R^4$ wie unter Formel (I) definiert sind, mit einem Alkylierungs- oder Acylierungsmittel der Formel $R^{2-a}W^1$ (IV), worin $W^1$ eine Leaving-Gruppe darstellt und $R^{2-a}$ für $C_{1-6}$-Alkyl; Hydroxy-$C_{1-6}$-alkyl; Phenyl, das gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist; Aryl-$C_{1-6}$-alkyl; $C_{1-6}$-Alkylcarbonyl; $C_{1-6}$-Alkyloxycarbonyl oder Phenylcarbonyl steht, worin das Phenyl gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist, durch Behandlung mit einer Base in einem reaktionsinerten Lösungsmittel, gegebenenfalls bei erhöhter Temperatur, unter Ausbildung einer Verbindung der Formel

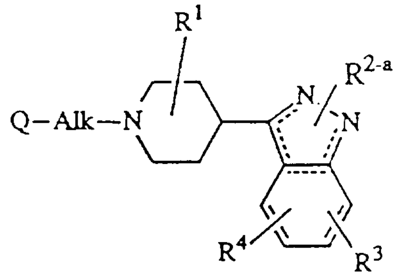

(I-a),

worin Q, Alk, $R^1$, $R^3$ und $R^4$ wie unter Formel (I) definiert sind und $R^{2-a}$ für $C_{1-6}$-Alkyl; Hydroxy-$C_{1-6}$-alkyl; Phenyl, das gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist; Aryl-$C_{1-6}$-alkyl; $C_{1-6}$-Alkylcarbonyl; $C_{1-6}$-Alkyloxycarbonyl oder Phenylcarbonyl steht, worin das Phenyl gegebenenfalls durch bis zu drei Substituenten, unabhängig ausgewählt unter $C_{1-6}$-Alkyl, $C_{1-6}$-Alkyloxy, Hydroxy, Halogen, Amino, Nitro und Trifluormethyl, substituiert ist;

c) Cyclisieren eines Zwischenprodukts der Formel

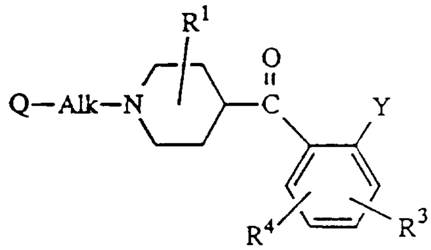

(V),

worin Q, Alk, $R^1$, $R^3$ und $R^4$ wie unter Formel (I) definiert sind und Y eine Leaving-Gruppe bezeichnet, mit einem Hydrazinderivat $R^2$-NH-$NH_2$ (VI) oder einem Säureadditionssalz hievon in einem reaktionsinerten Lösungsmittel in Anwesenheit einer Base, gewünschtenfalls bei erhöhter Temperatur;

und gewünschtenfalls Überführen der Verbindungen der Formel (I) in eine therapeutisch wirksame nicht-toxische Säureadditionssalzform durch Behandlung mit einer Säure; oder umgekehrt Überführen des Säuresalzes in die freie Base mit Alkali; und/oder Bereiten stereochemisch isomerer Formen hievon.

## Revendications

**Revendications pour les Etats contractants suivants : AT, BE, CH, DE, FR, GB, IT, LI, LU, NL, SE**

**1.** Composé chimique de formule

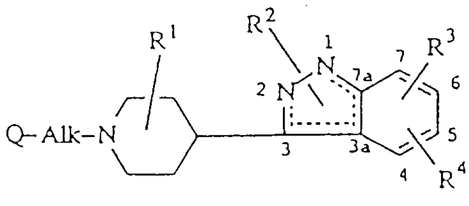

(I),

un de ses sels d'addition d'acides pharmaceutiquement acceptables ou formes stéréochimiquement isomériques, dans laquelle

$R^1$ représente un hydrogène ou un alkyle en $C_1$ à $C_6$;

$R^2$ est un hydrogène; un alkyle en $C_1$ à $C_6$, un hydroxyl-alkyle en $C_1$ à $C_6$; un phényle facultativement substitué par jusqu'à trois substituants choisis indépendamment parmi alkyle en $C_1$ à $C_6$, alkyloxy en $C_1$ à $C_6$, hydroxy, halo, amino, nitro et trifluorométhyle; aryl-alkyle en $C_1$ à $C_6$; alkyle

en $C_1$ à $C_6$-carbonyle; alkyloxy en $C_1$ à $C_6$ carbonyle ou phénylcarbonyle, où phényle est facultativement substitué par jusqu'à trois substituants choisis indépendamment parmi alkyle en $C_1$ à $C_6$, alkyloxy en $C_1$ à $C_6$, hydroxy, halo, amino, nitro et trifluorométhyle;

$R^3$ et $R^4$ représentent chacun indépendamment un hydrogène, halo, hydroxy, alkyloxy en $C_1$ à $C_6$ ou alkyle en $C_1$ à $C_6$;

Alk est un alcanediyle en $C_1$ à $C_4$ ; et

Q est un radical hétérocyclique bicyclique de formule

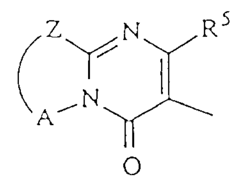

(a),

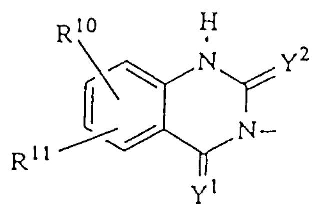

(b)

ou

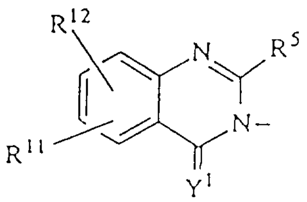

(c);

où

$R^5$ est un hydrogène ou un alkyle en $C_1$ à $C_6$ ;

Z représente -S- ou $-CR^6=CR^7-$; lesdits $R^6$ et $R^7$ représentant chacun indépendamment un hydrogène ou un alkyle en $C_1$ à $C_6$ ; ou Z représente $-CH_2-$ où un atome d'hydrogène peut être remplacé par un hydroxy ou un alkyle en $C_1$ à $C_6$ ;

A un radical bivalent $-CH_2-CH_2-$ ou $-CH_2CH_2-CH_2-$ où dans ces deux derniers radicaux, un ou deux atomes d'hydrogène peuvent être remplacés par un alkyle en $C_1$ à $C_6$ ; ou A est un radical bivalent $-CR^8=CR^9-$, où $R^8$ et $R^9$ représentent chacun indépendamment un hydrogène, halo, amino ou alkyle en $C_1$ à $C_6$ ; ou lorsque Z représente -S-, A peut également représenter $-CR^{10}=N-$, $R^{10}$ étant un hydrogène ou un alkyle en $C_1$ à $C_6$ ; ou lorsque Z représente $-CR^6=CR^7-$, A peut également représenter -O-; et

$Y^1$ et $Y^2$ représentent chacun indépendamment O ou S;

$R^{11}$ est un hydrogène, halo, alkyle en $C_1$ à $C_6$ , alkyloxy en $C_1$ à $C_6$, trifluorométhyle, nitro, mono- ou di(alkyle en $C_1$ à $C_6$)amino, alkyle en $C_1$ à $C_{10}$-carbonylamino, cyano, alkyle en $C_1$ à $C_{10}$-carbonyloxy, phénylméthoxy ou azido.

$R^{12}$ est un hydrogène ou un halo; et

aryle est un phényle facultativement substitué par jusqu'à trois substituants choisis indépendamment parmi alkyle en $C_1$ à $C_6$, alkyloxy en $C_1$ à $C_6$, hydroxy, halo, amino, nitro et trifluorométhyle; pyridinyle, furanyle ou furanyle substitué par un alkyle en $C_1$ à $C_6$.

**2.** Composé chimique selon la revendication 1 dans lequel $R^1$ est un hydrogène; $R^2$ est substitué sur $N^1$, $R^3$ et $R^4$ représentent indépendamment un hydrogène, un alkyloxy en $C_1$ à $C_6$ ou un halo; Q est un radical de formule (a) où $R^5$ est un alkyle en $C_1$ à $C_6$.

**3.** Composé chimique selon la revendication 1, où $R^2$ est un hydrogène, un alkyle en $C_1$ à $C_6$, un phényle facultativement substitué par un halo ou un trifluorométhyle, ou un phénylméthyle facultativement substitué par un halo, un alkyloxy en $C_1$ à $C_6$ ou un trifluorométhyle; $R^3$ est un halo; $R^4$ est un hydrogène.

**4.** Composé chimique selon la revendication 1, où $R^3$ est un 6-fluoro.

**5.** Composition pharmaceutique comprenant un support pharmaceutique approprié et comme ingrédient actif une quantité thérapeutiquement efficace d'un composé selon l'une quelconque des revendications 1 à 4.

**6.** Composition pharmaceutique anti-hypertensive comprenant un support pharmaceutique approprié et comme ingrédient actif une quantité hypertensive efficace d'un composé selon l'une quelconque des revendications 1 à 4.

**7.** Procédé de préparation d'une composition pharmaceutique selon les revendications 5 ou 6, caractérisé en ce qu'on mélange intimement une quantité thérapeutiquement efficace d'un composé tel que défini dans l'une quelconque des revendications 1 à 4 avec des supports pharmaceutiques appropriés.

**8.** Composé selon l'une quelconque des revendications 1 à 4, pour utilisation comme médicament.

**9.** Composé selon l'une quelconque des revendications 1 à 4, pour utilisation comme médicament antihypertensif.

**10.** Procédé de préparation d'un composé chimique selon l'une quelconque des revendications 1 à 4, caractérisé en ce que :

a) on N-alkyle une pipéridine de formule

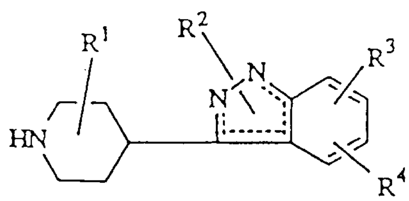

(III)

dans laquelle $R^1$, $R^2$, $R^3$ et $R^4$ sont tels que définis sous la formule (I) avec un réactif alkylant Q-Alk-W (II) dans lequel Q et Alk sont tels que définis sous la formule (I) et où W est un groupe sortant, dans un solvant inerte vis-à-vis de la réaction, facultativement en présence d'une base et facultativement en présence d'un iodure de métal alcalin, en agitant à une température élevée, ou

b) on N-alkyle ou N-acyle un composé de formule

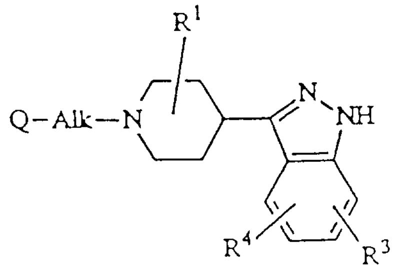

(I-b),

dans laquelle Q, Alk, $R^1$, $R^3$ et $R^4$ sont tels que défnis dans la formule (I), avec un réactif alkylant ou acylant de formule $R^{2-a}W^1$ (IV) dans laquelle $W^1$ est un groupe sortant et $R^{2-a}$ est un alkyle en $C_1$ à $C_6$, un hydroxyalkyle en $C_1$ à $C_6$; un phényle facultativement substitué par jusqu'à trois substituants choisis indépendamment parmi alkyle en $C_1$ à $C_6$, alkyloxy en $C_1$ à $C_6$, hydroxy, halo, amino, nitro

et trifluorométhyle; aryl-alkyle en $C_1$ à $C_6$; alkyle en $C_1$ à $C_6$-carbonyle; alkyloxy en $C_1$ à $C_6$-carbonyle ou phénylcarbonyle, où phényle est facultativement substitué par jusqu'à trois substituants choisis indépendamment parmi alkyle en $C_1$ à $C_6$, alkyloxy en $C_1$ à $C_6$ , hydroxy, halo, amino, nitro et trifluorométhyle, par traitement avec une base dans un solvant inerte vis-à-vis de la réaction, facultativement à une température élevée, donnant ainsi un composé de formule

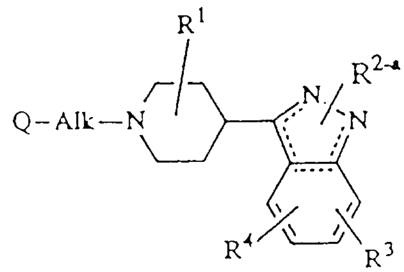

(I-a),

dans laquelle Q, Alk, $R^1$, $R^3$ et $R^4$ sont tels que définis sous la formule (I) et $R^{2-a}$ est un alkyle en $C_1$ à $C_6$ ; un hydroxy-alkyle en $C_1$ à $C_6$; un phényle facultativement substitué par jusqu'à trois substituants choisis indépendamment parmi alkyle en $C_1$ à $C_6$, alkyloxy en $C_1$ à $C_6$ , hydroxy, halo, amino, nitro et trifluorométhyle; aryl-alkyle en $C_1$ à $C_6$ ; alkyle en $C_1$ à $C_6$-carbonyle; alkyloxy en $C_1$ à $C_6$ -carbonyle ou phénylcarbonyle, où phényle est facultativement substitué par jusqu'à trois substituants choisis indépendamment parmi alkyle en $C_1$ à $C_6$ , alkyloxy en $C_1$ à $C_6$ , hydroxy, halo, amino, nitro et trifluorométhyle;

c) on cyclise un intermédiaire de formule

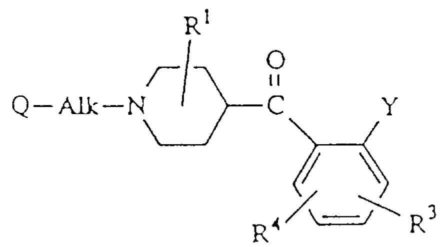

(V),

dans laquelle Q, Alk, $R^1$, $R^3$ et $R^4$ sont tels que définis sous la formule (I) et Y est un groupe sortant avec un dérivé d'hydrazine $R^2$-NH-$NH_2$ (VI) ou un de ses sels d'addition d'acides, dans un solvant inerte vis-à-vis de la réaction en présence d'une base, facultativement à une température élevée; et, si on le désire, on transforme les composés de formule (I) en une forme sel d'addition d'acide non toxique thérapeutiquement active par traitement avec un acide; ou inversement, on transforme le sel d'acide en la base libre avec un alcali; et/ou on prépare leurs formes stéréochimiquement isomériques.

**Revendication pour les Etats contractants suivants : ES, GR**

**1.** Procédé de préparation d'un composé chimique de formule

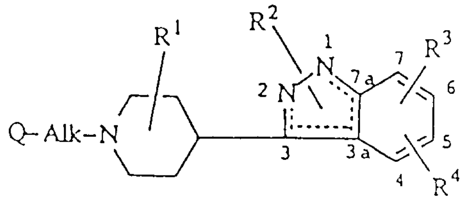

(I),

d'un de ses sels d'addition d'acides pharmaceutiquement acceptables ou formes stéréochimiquement isomériques, dans lequel

$R^1$ représente un hydrogène ou un alkyle en $C_1$ à $C_6$;

$R^2$ est un hydrogène; un alkyle en $C_1$ à $C_6$, un hydroxyl-alkyle en $C_1$ à $C_6$; un phényle facultativement substitué par jusqu'à trois substituants choisis indépendamment parmi alkyle en $C_1$ à $C_6$, alkyloxy en $C_1$ à $C_6$, hydroxy, halo, amino, nitro et trifluorométhyle; aryl-alkyle en $C_1$ à $C_6$; alkyle en $C_1$ à $C_6$-carbonyle; alkyloxy en $C_1$ à $C_6$ carbonyle ou phénylcarbonyle, où phényle est facultativement substitué par jusqu'à trois substituants choisis indépendamment parmi alkyle en $C_1$ à $C_6$, alkyloxy en $C_1$ à $C_6$, hydroxy, halo, amino, nitro et trifluorométhyle;

$R^3$ et $R^4$ représentent chacun indépendamment un hydrogène, halo, hydroxy, alkyloxy en $C_1$ à $C_6$ ou alkyle en $C_1$ à $C_6$;

Alk est un alcanediyle en $C_1$ à $C_4$ ; et

Q est un radical hétérocyclique bicyclique de formule

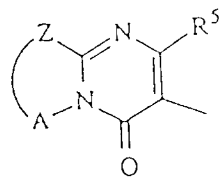

(a),

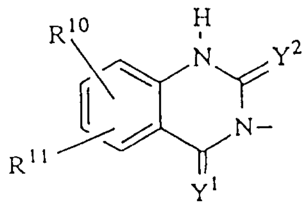

(b)

ou

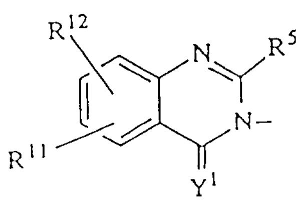

(c);

où

$R^5$ est un hydrogène ou un alkyle en $C_1$ à $C_6$ ;

Z représente -S- ou $-CR^6=CR^7-$; lesdits $R^6$ et $R^7$ représentant chacun indépendamment un hydrogène ou un alkyle en $C_1$ à $C_6$ ; ou Z représente $-CH_2-$ où un atome d'hydrogène peut être remplacé par un hydroxy ou un alkyle en $C_1$ à $C_6$ ;

A un radical bivalent $-CH_2-CH_2-$ ou $-CH_2CH_2-CH_2-$ où dans ces deux derniers radicaux, un ou deux atomes d'hydrogène peuvent être remplacés par un alkyle en $C_1$ à $C_6$ ; ou A est un radical bivalent $-CR^8=CR^9-$, où $R^8$ et $R^9$ représentent chacun indépendamment un hydrogène, halo, amino ou alkyle en $C_1$ à $C_6$ ; ou lorsque Z représente -S-, A peut également représenter $-CR^{10}=N-$, $R^{10}$ étant un hydrogène ou un alkyle en $C_1$ à $C_6$ ; ou lorsque Z représente $-CR^6=CR^7-$, A peut également représenter -O-; et

$Y^1$ et $Y^2$ représentent chacun indépendamment O ou S;

$R^{11}$ est un hydrogène, halo, alkyle en $C_1$ à $C_6$ , alkyloxy en $C_1$ à $C_6$, trifluorométhyle, nitro, amino, mono- ou di(alkyle en $C_1$ à $C_6$)amino, alkyle en $C_1$ à $C_{10}$-carbonylamino, cyano, hydroxy, alkyle en $C_1$ à $C_{10}$-carbonyloxy, phénylméthoxy ou azido.

$R^{12}$ est un hydrogène ou un halo; et

aryle est un phényle facultativement substitué par jusqu'à trois substituants choisis indépendam-

ment parmi alkyle en $C_1$ à $C_6$, alkyloxy en $C_1$ à $C_6$, hydroxy, halo, amino, nitro et trifluorométhyle; pyridinyle, furanyle ou furanyle substitué par un alkyle en $C_1$ à $C_6$, caractérisé en ce que

a) on N-alkyle une pipéridine de formule

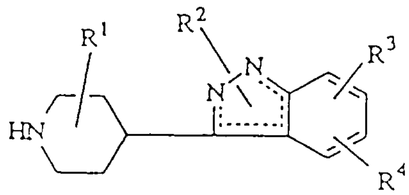

(III)

dans laquelle $R^1$, $R^2$, $R^3$ et $R^4$ sont tels que définis sous la formule (I) avec un réactif alkylant Q-Alk-W (II) dans lequel Q et Alk sont tels que définis sous la formule (I) et où W est un groupe sortant, dans un solvant inerte vis-à-vis de la réaction, facultativement en présence d'une base et facultativement en présence d'un iodure de métal alcalin, en agitant à une température élevée,

ou

b) on N-alkyle ou N-acyle un composé de formule

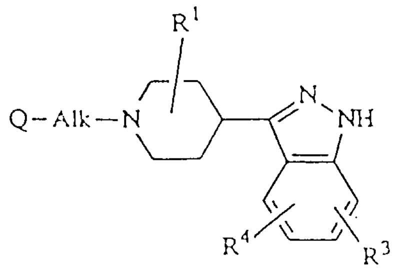

(I-b),

dans laquelle Q, Alk, $R^1$, $R^3$ et $R^4$ sont tels que défnis dans la formule (I), avec un réactif alkylant ou acylant de formule $R^{2-a}W^1$ (IV) dans laquelle $W^1$ est un groupe sortant et $R^{2-a}$ est un alkyle en $C_1$ à $C_6$, un hydroxyalkyle en $C_1$ à $C_6$; un phényle facultativement substitué par jusqu'à trois substituants choisis indépendamment parmi alkyle en $C_1$ à $C_6$, alkyloxy en $C_1$ à $C_6$, hydroxy, halo, amino, nitro et trifluorométhyle; aryl-alkyle en $C_1$ à $C_6$; alkyle en $C_1$ à $C_6$-carbonyle; alkyloxy en $C_1$ à $C_6$-carbonyle ou phénylcarbonyle, où phényle est facultativement substitué par jusqu'à trois substituants choisis indépendamment parmi alkyle en $C_1$ à $C_6$, alkyloxy en $C_1$ à $C_6$ , hydroxy, halo, amino, nitro et trifluorométhyle, par traitement avec une base dans un solvant inerte vis-à-vis de la réaction, facultativement à une température élevée, donnant ainsi un composé de formule

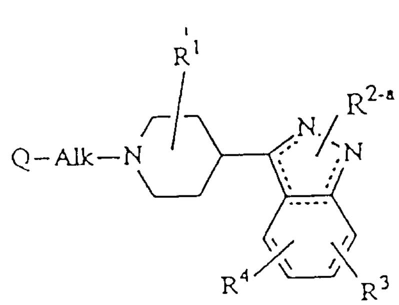

(I-a),

dans laquelle Q, Alk, $R^1$, $R^3$ et $R^4$ sont tels que définis sous la formule (I) et $R^{2-a}$ est un alkyle en $C_1$ à $C_6$ ; un hydroxy-alkyle en $C_1$ à $C_6$; un phényle facultativement substitué par jusqu'à trois substituants choisis indépendamment parmi alkyle en $C_1$ à $C_6$, alkyloxy en $C_1$ à $C_6$ , hydroxy, halo, amino, nitro et trifluorométhyle; aryl-alkyle en $C_1$ à $C_6$ ; alkyle en $C_1$ à $C_6$-carbonyle; alkyloxy en $C_1$ à $C_6$ -carbonyle ou phénylcarbonyle, où phényle est facultativement substitué par jusqu'à trois substituants choisis indépendamment parmi alkyle en $C_1$ à $C_6$ , alkyloxy en $C_1$ à $C_6$ , hydroxy, halo,

amino, nitro et trifluorométhyle;

c) on cyclise un intermédiaire de formule

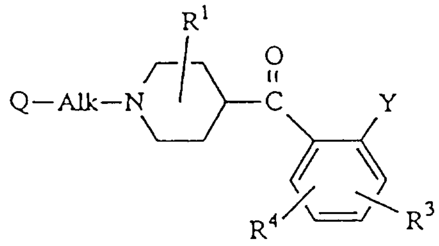

(V),

dans laquelle Q, Alk, $R^1$, $R^3$ et $R^4$ sont tels que définis sous la formule (I) et Y est un groupe sortant avec un dérivé d'hydrazine $R^2$-NH-$NH_2$ (VI) ou un de ses sels d'addition d'acides, dans un solvant inerte vis-à-vis de la réaction en présence d'une base, facultativement à une température élevée; et, si on le désire, on transforme les composés de formule (I) en une forme sel d'addition d'acide non toxique thérapeutiquement active par traitement avec un acide; ou inversement, on transforme le sel d'acide en la base libre avec un alcali; et/ou on prépare leurs formes stéréochimiquement isomériques.